(19) Europäisches Patentamt
European Patent Office
Office européen des brevets

(11) EP 0 656 353 B1

(12) EUROPÄISCHE PATENTSCHRIFT

(45) Veröffentlichungstag und Bekanntmachung des Hinweises auf die Patenterteilung: **03.06.1998 Patentblatt 1998/23**

(51) Int. Cl.[6]: **C07D 215/42**, A61K 31/47, C07D 401/12

(21) Anmeldenummer: **94116281.0**

(22) Anmeldetag: **15.10.1994**

# (54) Aminochinolin-Derivate mit einer Wirksamkeit gegen Malariaerreger

Aminochinoline derivates useful in the treatment of malaria

Dérivés d'aminoquinoleines utiles pour le traitement de la malaria

(84) Benannte Vertragsstaaten:
**AT BE CH DE DK ES FR GB GR IE IT LI LU NL PT SE**

(30) Priorität: **28.10.1993 3255/93**

(43) Veröffentlichungstag der Anmeldung: **07.06.1995 Patentblatt 1995/23**

(73) Patentinhaber:
**F. HOFFMANN-LA ROCHE AG**
**4002 Basel (CH)**

(72) Erfinder:
- **Hofheinz, Werner**
  **CH-4103 Bottmingen (CH)**
- **Jaquet, Catherine**
  **CH-4052 Basel (CH)**
- **Jolidon, Synèse**
  **CH-4223 Blauen (CH)**

(74) Vertreter: **Poppe, Regina et al**
**F.Hoffmann-La Roche AG**
**Patent Department(PLP),**
**124 Grenzacherstrasse**
**4070 Basel (CH)**

(56) Entgegenhaltungen:
**EP-A- 0 056 766**
**WO-A-93/07126**
**US-A- 3 184 462**

- **J.MED.CHEM., Bd.35, 1992 Seiten 2129 - 2134 VENNERSTROM ET AL 'BISQUINOLINES'**
- **J.MED.CHEM., 1971 14(4) pp283-6**
- **Beilstein EIII7IV 22, Syst. Nr. 3396/H445**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.3/3.4

**Beschreibung**

Die Erfindung betrifft Aminochinolin-Derivate der allgemeinen Formel

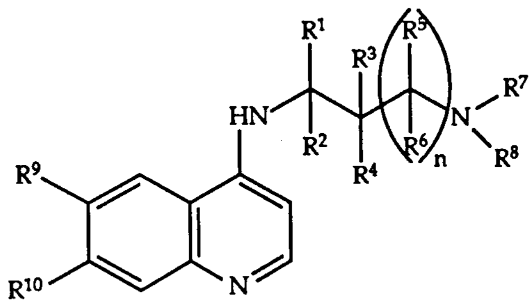

I

worin die Symbole $R^1$ bis $R^6$ Wasserstoff oder eines oder zwei davon $(C_1\text{-}C_4)$-Alkyl und die anderen Wasserstoff, $R^7$ und $R^8$ $(C_1\text{-}C_4)$-Alkyl, $(C_2\text{-}C_4)$-Alkenyl, Aryl-$(C_1\text{-}C_4)$-Alkyl oder mit N zusammen Pyrrolidin oder Piperidin, auch substituiert durch $(C_1\text{-}C_4)$-Alkyl, Octahydroindol oder 3-Azabicyclo[3,2,2] nonan und n= 0 oder 1 oder
worin die Symbole $R^1$ und $R^3$ Tri- oder Tetramethylen, alle übrigen Substituenten bis $R^6$ Wasserstoff, n = 0 und $R^7$ und $R^8$ die obigen Bedeutungen besitzen oder
worin die Symbole $R^1$ und $R^7$ Methylen oder Dimethylen und n = 1,

$R^1$ und $R^7$ Di- oder Trimethylen und n = 0,
$R^3$ und $R^7$ Di- oder Trimethylen und n = 1
$R^3$ und $R^7$ Tri- oder Tetramethylen und n = 0
$R^5$ und $R^7$ Tri- oder Tetramethylen und n = 1
$R^1$ und $R^5$ Di- oder Trimethylen und n = 1, alle übrigen Substituenten Wasserstoff, ausser $R^8$, das $(C_1\text{-}C_4)$-Alkyl, $C_2\text{-}C_4)$-Alkenyl oder Aryl-$(C_1\text{-}C_4)$-Alkyl bedeutet oder
worin die Symbole $R^3$ und $R^5$ Tri- oder Tetramethylen und n = 1, alle übrigen Substituenten bis $R^6$ Wasserstoff und $R^7$ und $R^8$ $(C_1\text{-}C_4)$-Alkyl, $(C_2\text{-}C_4)$-Alkenyl, Aryl-$(C_1\text{-}C_4)$-Alkyl oder mit N zusammen Pyrrolidin oder Piperidin, auch substituiert durch $(C_1\text{-}C_4)$-Alkyl,
$R^9$ Wasserstoff oder Halogen und $R^{10}$ Halogen oder Trifluormethyl bedeuten, sowie pharmazeutisch annehmbare Salze von basischen Verbindungen der allgemeinen Formel I.

Diese Verbindungen besitzen die überraschende Eigenschaft, dass sie sowohl gegen chloroquin-sensitive als auch gegen cbloroquin-resistente Malariaerreger eine gleich gute Wirkung haben, das heisst, dass sie keine Kreuzresistenz mit Chloroquin zeigen.
Sowohl die fehlende Kreuzresistenz als auch die gute, im Vergleich zu Chloroquin teilweise bessere Wirksamkeit waren überraschend. Bisher war angenommen worden, dass zwischen Chloroquin-ähnlichen Verbindungen Kreuzresistenz besteht. Einzig gewisse Bis-chinoline, welche zwei Chinolin-Ringe enthalten und dem Chloroquin strukturell weniger nahestehen, besitzten eine gewisse Wirksamkeit gegen chloroquin-resistente Malariaerreger, insbesondere die Verbindungen, die von J.L.Vennerstrom beschrieben worden sind (J. L. Vennerstrom et al, J.Med.Chem., *35*, 2129 - 2134 (1992). Ferner galt bisher die Annahme, dass Chloroquin-Analoga mit verkürzter oder verlängerter Seitenkette im Vergleich zu Chloroquin geringere Wirksamkeit gegen Malariaerreger besitzen (R.L.O'Brien und F.E.Hahn, Chloroquin Structural Requirements for Binding to Deoxyribonucleic Acid and Antimalarial Activity, Antimicrob. Agents Chemother, 1965, 315 - 320).

In der EP 0 056 766 wird ein Verfahren zur Herstellung von 4-Aminochlorchinolinen beschrieben, wobei für die hergestellten chloroquin-analogen Verbindungen eine Wirksamkeit gegen Malaria erwähnt wird.

Einfache Analoga des Chloroquins galten bisher als uninteressant und nicht geeignet für die Behandlung der Malaria. Im Gegensatz zu dieser Annahme hat sich nun gezeigt, dass sich die Verbindungen der Formel I hervorragend zur Vorbeugung gegen die Malaria und zu deren Behandlung eignen, insbesondere dort, wo die Erreger gegen Chloroquin resistent sind.

Gegenstand der vorliegenden Erfindung ist die Verwendung von Verbindungen der Formel I und von pharmazeutisch anwendbaren Salzen davon bei der Bekämpfung oder Verhütung von Krankheiten, insbesondere bei der Bekämpfung von sowohl chloroquin-resistenten als auch von chloroquin-sensitiven Malariaerregern, die neuen Verbindungen und Salze der Formel I als solche und zur Anwendung als therapeutische Wirkstoffe, die Herstellung der neuen Verbindungen und Salze sowie Arzneimittel, enthaltend eine neue Verbindung oder ein Salz und die Herstellung solcher Arzneimittel.

Der in der vorliegenden Beschreibung verwendete Ausdruck "Alkyl" bezeichnet geradkettige oder verzweigte gesättigte Kohlenwasserstoffreste, wie Methyl, Aethyl, n-Propyl, Isopropyl, n-Butyl, Isobutyl, s-Butyl, t-Butyl und dergleichen. Halogen bedeutet Chlor, Brom, Fluor oder Jod.

Besonders bevorzugt ist die Verwendung von Verbindungen, worin die Symbole $R^1$ bis $R^6$ Wasserstoff oder eines oder zwei davon ($C_1$-$C_4$)-Alkyl, und die anderen Wasserstoff, $R^7$ und $R^8$ ($C_1$-$C_4$)-Alkyl, ($C_2$-$C_4$)-Alkenyl, Aryl-($C_1$-$C_4$)-Alkyl oder mit N zusammen Pyrrolidin oder Piperidin, auch substituiert durch ($C_1$-$C_4$)-Alkyl, und n = 0 oder 1 bedeuten.

Ganz besonders bevorzugte Verbindungen der allgemeinen Formel I für die oben genannte Verwendung sind:

(S)-$N_2$-(7-Chlor-chinolin-4-yl)-$N_1$,$N_1$-dimethyl-propan-1,2-diamin,

(R)-$N_2$-(7-Chlor-chinolin-4-yl)-$N_1$,$N_1$-dimethyl-propan-1,2-diamin,

$N_1$-(7-Chlor-chinolin-4-yl)-2,$N_2$,$N_2$-trimethyl-propan-1,2-diamin,

$N_3$-(7-Chlor-chinolin-4-yl)-$N_1$,$N_1$-diethyl-propan-1,3-diamin,

(RS)-(7-Chlor-chinolin-4-yl)-(1-methyl-piperidin-3-yl)amin und

(RS)-(7-Chlor-chinolin-4-yl)-(1-methyl-pyrrolidin-3-yl)-amin.

Weiterhin bevorzugt sind:

(RS)-$N_2$-(7-Chlor-chinolin-4-yl)-$N_1$,$N_1$-dimethyl-propan-1,2-diamin,

(RS)-$N_2$-(7-Chlor-chinolin-4-yl)-$N_1$,$N_1$-diethyl-propan-1,2-diamin,

(S)-$N_2$-(7-Chlor-chinolin-4-yl)-$N_1$,$N_1$-diethyl-propan-1,2-diamin,

(R)-$N_2$-(7-Chlor-chinolin-4-yl)-$N_1$,$N_1$-diethyl-propan-1,2-diamin,

(RS)-(7-Chlor-chinolin-4-yl)-(1-methyl-2-pyrrolidin-1-yl-ethyl)-amin,

$N_2$-(7-Chlor-chinolin-4-yl)-$N_1$,$N_1$-dimethyl-ethan-1,2-diamin,

$N_2$-(7-Chlor-chinolin-4-yl)-$N_1$,$N_1$-diethyl-ethan-1,2-diamin,

$N_3$-(7-Chlor-chinolin-4-yl)-$N_1$,$N_1$-dimethyl-propan-1,3-diamin,

(R)-$N_1$-(7-Chlor-chinolin-4-yl)-$N_2$,$N_2$-dimethyl-propan-1,2-diamin,

(S)-$N_1$-(7-Chlor-chinolin-4-yl)-$N_2$,$N_2$-dimethyl-propan-1,2-diamin und

(RS)-(7-Chlor-chinolin-4-yl)-( 1-methyl-pyrrolidin-2-yl-methyl)-amin.

Die Erfindung betrifft auch neue Verbindungen der Formel I, worin $R^7$ und $R^8$($C_2$-$C_4$)-Alkenyl, Aryl-($C_1$-$C_4$)-Alkyl, Octahydroindolyl oder 3-Azabicyclo[3,2,2]nonan und alle anderen Substituenten wie oben beschrieben bedeuten, und die man erfindungsgemäss herstellen kann, indem man

a) Chinolinderivate der allgemeinen Formel

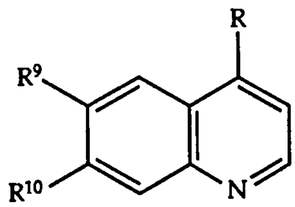

II

worin $R^9$ und $R^{10}$ die obigen Bedeutungen haben und R eine Abgangsgruppe bedeutet, mit Aminoverbindungen der allgemeinen Formel

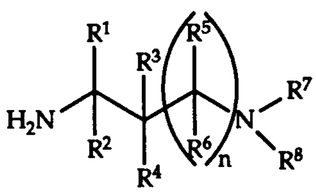

III

worin die Substituenten $R^1$ bis $R^8$ obige Bedeutungen haben, umsetzt, oder

b) Alkylamino-chinolinderivate der allgemeinen Formel

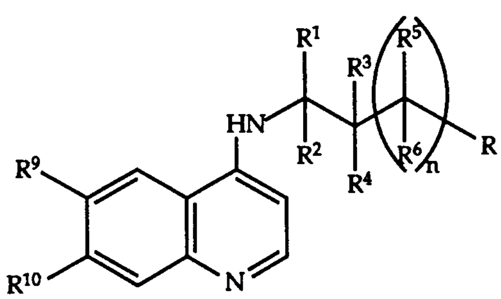

IV

worin $R^1$ bis $R^6$ und $R^9$ und $R^{10}$ obige Bedeutungen haben und R eine Abgangsgruppe bedeutet, mit Aminen der Formel

$$HNR^7R^8 \quad V$$

worin $R^7$ und $R^8$ obige Bedeutungen haben, umsetzt, und

*c)* erwünschtenfalls eine basische Verbindung der Formel I mittels einer Säure in ein pharmazeutisch anwendbares Salz überführt.

Gemäss Verfahrensvariante a) des erfindungsgemässen Verfahrens werden entsprechend substituierte Chinolinderivate, die in 4-Stellung eine Abgangsgruppe enthalten, mit Aminoverbindungen der allgemeinen Formel III umgesetzt, wobei die Substituenten die oben angegebenen Bedeutungen haben und R eine Abgangsgruppe bedeutet.

Abgangsgruppen können zweckmässigerweise Halogen-, O-Methylsulfonyl- oder O-Toluolsulfonylgruppen sein. Die Umsetzung erfolgt zweckmässigerweise in einem Temperaturbereich zwischen 120 und 180°C und in einem Lösungsmittel, wobei Phenol, Ethoxyethanol, Dimethylacetamid oder N-Methylpyrrolidin besonders bevorzugt sind. Die Reaktionsdauer kann zwischen 2 und 28 Stunden variieren.

Eine weitere Möglichkeit der Herstellung der Verbindungen der allgemeinen Formel I besteht in der Anwendung der Verfahrensvariante b).

Zweckmässigerweise werden die Verbindungen der Formel IV, worin R eine Abgangsgruppe bedeutet und alle anderen Substituenten den oben genannten Bedeutungen entsprechen, in Form des Hydrochlorids des entsprechend substituierten Alkylamino-chinolinderivates mit einem aliphatischen oder cyclischen Amin der Formel V in einem geschlossenen Rohr, wobei das Amin gleichzeitig als Lösungsmittel dienen kann, umgesetzt. Die Reaktion kann bis zu

24 h dauern. Der bevorzugte Temperaturbereich umfasst Temperaturen zwischen 90 und 110°C. Diese Umsetzung kann auch in Lösungsmitteln erfolgen, in denen beide Reaktionspartner löslich sind, beispielsweise in DMF, DMA, N-Methylpyrrolidon oder Acetonitril. Die Ueberführung in ein pharmazeutisch anwendbares Salz erfolgt durch Zuführung einer Säure. Wegen der physiologischen Verträglichkeit des Hydrochlorids ist HCl besonders bevorzugt. Als Lösungsmittel sind zweckmässigerweise besonders geeignet: Isopropanol, Diethylether oder Dioxan.

Die für die Synthesevariante a) benötigten Chinolin-Derivate der allgemeinen Formel II sind Handelsprodukte oder können nach an sich bekannte Methoden hergestellt werden, beispielsweise kann man 7-Chlor-4-hydroxychinolin mit Phosphoroxybromid zu 7-Chlor-4-brom-chinolin umsetzen.

Die aliphatischen oder cyclischen Amine der allgemeinen Formel III sind ebenso teilweise Handelsprodukte oder können nach an sich bekannten Methoden hergestellt werden. Zweckmässigerweise kann man von einem cyclischen Amin, beispielsweise Pyrrolidin oder Piperidin, oder von einem aliphatischen Amin, z.B. Dimethylamin oder Diethylamin, ausgehen und mit Nitroethan oder 2-Nitropropan umsetzen. Die entstandene Nitro-Verbindung kann anschliessend nach an sich bekannten Methoden in die Verbindungen der allgemeinen Formel III reduziert werden, z.B. durch Hydrierung mit Raney-Nickel.

Eine weitere Möglichkeit der Herstellung der Verbindungen der allgemeinen Formel III besteht darin, Oxide von Cycloalkanen mit Diethylamin und Perchloraten zu den 2-(Diethylamio)-cycloalkanolen umzusetzen. Diese werden zweckmässigerweise ohne weitere Reinigung in THF gelöst und mit Phthalimid und Triphenylphosphin versetzt und anschliessend mehrere Stunden bei Raumtemperatur gerührt. Das entstandene N,N-Diethyl-2-phthalimido-cycloalkylamin wird nach Reinigung mit konzentrierter Salzsäure verseift.

In Analogie dazu kann man anstelle von Diethylamin und anderen aliphatischen Aminen auch beispielsweise cyclische Amine, wie Pyrrolidin, verwenden. Setzt man cycliche Amine, beispielsweise Piperidin, mit Acrylnitril um, erhält man die entsprechenden Propionitrile, die in Gegenwart von Platindioxid bei RT unter 10 bar in die entsprechenden Propylamine hydriert werden können.

Das als Ausgangsstoff für die Synthesevariante b) benötigte entsprechende Alkylamino-chinolinderivat der allgemeinen Formel IV, worin die Abgangsgruppe eine Halogengruppe, vorzugsweise Chlor, bedeutet, kann zweckmässigerweise folgendermassen hergestellt werden:

Eine Suspension des entsprechenden Chinolinamino-Ethanols, das nach einer Synthese von R.C. Elderfield, J. Am. Chem. Soc. 68, 1250 (1946) hergestellt werden kann, versetzt man mit Thionylchlorid, wobei die Reaktionstemperatur 30°C nicht überschreiten sollte. Anschliessend wird etwa noch 1 h bei RT nachgerührt, zur Trockne eingedampft und gereinigt.

Wie eingangs erwähnt, haben die Aminochinolin-Derivate der allgemeinen Formel I und deren pharmazeutisch anwendbaren Salze äusserst wertvolle pharmakologische Eigenschaften.

Ihre Wirkung gegen sowohl chloroquin-resistente als auch chloroquinsensitive Malariaerreger zeigen die folgen Tabellen:

Testmethode für die Bestimmung der Aktivität gegen P.falciparum in vitro

Die Präparate werden an intraerythrozytären Stadien von P. falciparum aus asynchronen Kulturen nach der Methode von Desjardin et al getestet. (Desjardins, R.E. et al: Quantitative assessment of antimalarial activity in vitro by a semiautomated microdilution technique. Antimicrob. Agents Chemother. 16, 710-718, (1979)).

Das Kulturmedium besteht aus RPMI 1640 mit Zusatz von 25 mM HEPES, 25 mM $NaHCO_3$, 100 µg/ml Neomycin und 10% Humanserum ($A^+$). Als Wirtszellen von P. falciparum dienen Human-$A^+$-Erythrozyten. Die Parasiten werden in einer Atmosphäre von 3% $O_2$, 4% $CO_2$, 93% $N_2$ und 95% relativer Luftfeuchtigkeit bei 37°C gehalten.

Zur Aktivitätsbestimmung werden die Präparate in DMSO gelöst, im Kulturmedium bis zu einer geeigneten Ausgangskonzentration vorverdünnt und anschliessend in 2er-Schritten über 6-7 Stufen in Mikrotiterplatten austitriert. Nach Zugabe der Parasitenkultur (0.7% Parasitämie in 2.5%iger Erythrozytensuspension) werden die Testplatten unter den oben angegebenen Bedingungen während 48 h - 72 h inkubiert. Das Wachstum der Parasiten bei den verschiedenen Präparatekonzentrationen wird mittels Inkorporation von [G-$^3$H]-Hypoxanthin im Vergleich zu unbehandelten Kontrollkulturen auf derselben Testplatte bestimmt. Aus der daraus resultierenden Dosis-Wirkungskurve wird die 50%ige Wachstumshemmung (IC50) nach Logit-Regressionsanalyse berechnet.

Die Präparate werden an mindestens einem chloroquin-resistenten und einem chloroquin-sensitiven Stamm von P. falciparum geprüft. Zur weiteren Charakterisierung werden zusätzliche sensitive und resistente Stämme einbezogen.

Testmethode für die Bestimmung der Aktivität gegen Plasmodium berghei in vivo.

Die Präparate werden an Mäusen getestet, die mit Malariaerregern (Plasmodium berghei) infiziert sind. Als Versuchstiere verwendet man männliche, ca 25 g schwere Albinomäuse (IBM:MORO(SPF), FUELLINSDORF). Sie werden in klimatisierten Räumen bei 21-22°C gehalten, und zwar in Gruppen von 5 Tieren pro Käfig. Sie erhalten ad libitum

ein Diätfutter mit geringem PABA-Gehalt (NAFAG FUTTER © No. 9009 PAB-45, PABA Gehalt 45 mg/kg) und Trinkwasser. Am ersten Versuchstag (DO) werden die Versuchstiere mit Plasmodium berghei (Stamm ANKA) infiziert. Dazu dient heparinisiertes Blut einer Spendermaus mit ca. 30% Parasitaemie, das mit physiologischer Kochsalzlösung so verdünnt wird, dass es pro ml $10^8$ parasitierte Erythrozyten enthält. 0.2 ml dieser Suspension werden intravenös (i.v.) den zu behandelnden Mäusen sowie den Kontrollmäusen injiziert. In unbehandelten Kontrolltieren erreicht die Parasitaemie am dritten Tag nach der Infektion (D+3) regelmässig 30-40%, und die Versuchstiere sterben zwischen den Tagen +5 und +7.

Die zu testenden Substanzen werden in destilliertem Wasser oder in einer Mischung aus 7% Tween-80, 3% Alkohol (96%) und Wasser gelöst oder suspendiert. In der Regel werden je 0.25 ml dieser Lösung oder Suspension subkutan und peroral an Gruppen von 5 Versuchstieren einmalig verabreicht. Die Behandlung erfolgt 24 Stunden nach der Infektion. Pro Versuch werden 10 Kontrolltiere in gleicher Weise mit Lösungs- oder Suspensions-medium behandelt.

Alle Substanzen werden in einem ersten Versuch in einer einmaligen Dosis von 10 mg/kg getestet. Zur Titration kommen nur diejenigen Substanzen, welche in diesem Versuch (10 mg/kg) eine Reduktion der Parasitaemie von 90% gezeigt haben. Zur genauen Titration der Wirkung kommen geeignete Verdünnungen der Testsubstanz zur Anwendung.

48 Stunden nach der Behandlung (D+3) werden von allen Tieren Blutausstriche mit Blut aus Schwanzvenen angefertigt und mit Giemsa gefärbt. Die mittlere Erythrozyten-Infektionsrate (Parasitaemie in %) in der Kontrollgruppe sowie in den Gruppen, welche mit den zu testenden Verbindungen behandelt worden sind, wird durch Auszählen unter dem Mikroskop bestimmt. Die Differenz des Mittelwertes der Infektionsrate von Kontrollgruppe (100%) und behandelten Gruppen wird errechnet und als Prozent Reduktion (GI%) ausgedrückt. Die ED50 oder ED90 wird rechnerisch mittels des JMP-Programmes (nonlinear-fit) ermittelt. Die ED50 (ED90) in mg/kg ist diejenige Dosis, welche nach einmaliger Verabreichung die mittlere Erythrozyten-Infektionsrate im Vergleich zur Kontrollgruppe um 50% (90%) reduziert.

Tabelle 1 enthält die in vitro gemessenen IC50 Werte für die Wachstumshemmung chloroquin-sensitiver und chloroquin-resistenter Stämme des human-pathogenen Plasmodium falciparum.

Tabelle 2 enthält Daten über die in vivo gegen Plasmodium-berghei an der Maus gemessene Aktivität: GI % ist die prozentuale Reduktion der Parasitaemie nach einer einmaligen, peroral (po) oder subcutan (sc) verabreichten Dosis von 10 mg/kg der Testsubstanz; ED50 und ED90 sind die wirksamen, peroral oder subcutan verabreichten Dosen der Testsubstanz.

Tabelle 1

| Beispiel Nr. | Chloroquin-sensitive Stämme, IC50 (ng/ml) | | | | | Chloroquin-resistente Stämme, IC50 (ng/ml) | | | | | | |
|---|---|---|---|---|---|---|---|---|---|---|---|---|
| | NF54 | FCH5C2 | HB3 | RFMEF3 | Ro73 | RFCR3 | ItG2 F6 | Indo | K1 | W2 | 7G8 | W2 Mef |
| 1 | 4 | 8 | 7 | 7 | 5 | 15 | 4 | 8 | 9 | 9 | 9 | 7 |
| 2 | 7 | 7 | 7 | 7 | 6 | 8 | 5 | 6 | 14 | 8 | 8 | 9 |
| 3 | 7 | 7 | 5 | 7 | 4 | 8 | 5 | 6 | 12 | 7 | 7 | 11 |
| 4 | 7 | 8 | 8 | 7 | 4 | 9 | 5 | 8 | 15 | 15 | 7 | 14 |
| 5 | 4 | 9 | 6 | | | 6 | 4 | 4 | 7 | 7 | 7 | 9 |
| 6 | 5 | 8 | 8 | | | 8 | 5 | 4 | 9 | 8 | 9 | 8 |
| 7 | 4 | 7 | 6 | | | 7 | 5 | 4 | 8 | 7 | 8 | 8 |
| 8 | 5 | 9 | 7 | 9 | 4 | 10 | 4 | 5 | 11 | 11 | 7 | 9 |
| 9 | 11 | 15 | 14 | 12 | 11 | 17 | 17 | 18 | 32 | 31 | 30 | 25 |
| 10 | 7 | 5 | 8 | 6 | 7 | 18 | 7 | 16 | 17 | 13 | 15 | 15 |
| 11 | 5 | 7 | 6 | 6 | 5 | 13 | 6 | 10 | 18 | 15 | 9 | 9 |
| 11a | 7 | 7 | 6 | 7 | 4 | 18 | 8 | 15 | 15 | 17 | 14 | 15 |
| 12 | 6 | 9 | 7 | 8 | 7 | 15 | 8 | 12 | 10 | 15 | 15 | 9 |
| 12a | 7 | 8 | 10 | 8 | 7 | 11 | 5 | 11 | 9 | 9 | 8 | 6 |
| 13 | 30 | 28 | 38 | 27 | 30 | 58 | 25 | 40 | 47 | 34 | 39 | 41 |
| 14 | 9 | 8 | 9 | 6 | 4 | 8 | 6 | 10 | 21 | 20 | 7 | 14 |
| 15 | 2 | 5 | 3 | | | 5 | 3 | 3 | 6 | 7 | 7 | 12 |
| 16 | 3 | 6 | 5 | | | 8 | 5 | 4 | 9 | 9 | 9 | 9 |
| 17 | 3 | 4 | 6 | 8 | 6 | 11 | 7 | 7 | 15 | 14 | 14 | 14 |
| 18 | 4 | 4 | 8 | 8 | 7 | 8 | 7 | 7 | 14 | 11 | 12 | 12 |
| 19 | 7 | 5 | 4 | 5 | 3 | 10 | 6 | 7 | 9 | 10 | 7 | 11 |
| 19a | 6 | 7 | 6 | 7 | 5 | 11 | 5 | 8 | 9 | 10 | 8 | 6 |
| 20 | 3 | 7 | 4 | 4 | 7 | 14 | 7 | 8 | 10 | 11 | 15 | 14 |
| 21 | 21 | 35 | 33 | 35 | 28 | 54 | 28 | 31 | 34 | 57 | 49 | |
| 22 | 14 | 17 | 20 | 18 | 11 | 20 | 14 | 16 | 22 | 11 | 29 | 23 |
| 23 | 6 | 6 | 7 | 7 | 4 | 10 | 6 | 8 | 15 | 15 | 8 | 13 |
| 24 | 7 | 7 | 7 | 7 | 4 | 9 | 6 | 8 | 15 | 15 | 7 | 9 |
| 25 | 8 | 8 | 10 | 8 | 5 | 14 | 8 | 15 | 22 | 26 | 11 | 16 |
| 26 | 7 | 8 | 8 | 9 | 5 | 8 | 6 | 9 | 15 | 15 | 8 | 13 |
| 27 | 8 | 10 | 9 | 7 | 3 | 15 | 7 | 9 | 18 | 19 | 7 | 14 |
| 28 | 7 | 14 | 11 | 9 | 5 | 16 | 6 | 9 | 16 | 18 | 9 | 15 |
| 29 | 6 | 6 | 7 | 5 | 3 | 11 | 6 | 7 | 16 | 15 | 5 | 11 |
| 30 | 7 | 9 | 10 | 8 | 11 | 16 | 7 | 7 | 14 | 15 | 15 | 8 |
| 31 | 5 | 5 | 5 | 6 | 5 | 14 | 7 | 7 | 11 | 14 | 9 | 7 |
| 32 | 7 | 9 | 9 | 8 | 7 | 16 | 10 | 15 | 16 | 15 | 15 | 13 |
| 33 | 8 | 15 | 15 | 16 | 10 | 18 | 9 | 14 | 14 | 21 | 18 | 15 |
| 33a | 7 | 8 | 7 | 7 | 5 | 9 | 5 | 9 | 8 | 9 | 9 | 7 |
| 33b | 8 | 11 | 12 | 8 | 4 | 10 | 17 | 9 | 17 | 9 | 18 | 18 |
| 33c | 8 | | | | | | | | 24 | | | |
| 33d | 8 | | | | | | | | 23 | | | |
| 33e | 12 | | | | | | | | 53 | | | |
| 33f | 12 | | | | | | | | 41 | | | |
| 33g | 5 | | | | | | | | 17 | | | |
| 33h | 7 | | | | | | | | 18 | | | |
| Chloroquin-diphosphat | 8 | 12 | 11 | 14 | 8 | 130 | 68 | 52 | 114 | 123 | 81 | 79 |

Tabelle 2

| Aktivität in vivo | | | |
|---|---|---|---|
| Beispiel Nr | GI % bei 10 mg/kg po | GI % bei 10 mg/kg sc | ED50 mg/kg po |
| 1 | 99.9 | 99.9 | 2.3 |
| 2 | 99.8 | 99.9 | 3.2 |
| 5 | 99.5 | 99.0 | 3.3 |
| 6 | 99.0 | 99.0 | 3.5 |
| 9 | 99.0 | 99.6 | 4.4 |
| 10 | 99.0 | 99.0 | 6.4 |
| 11 | 99.9 | 99.9 | 2.2 |
| 11a | 99.0 | 99.6 | 4.5 |
| 12a | 91.0 | 91.0 | 4.8 |
| 15 | 99.8 | 99.9 | 2.0 |
| 16 | 99.9 | 99.9 | 2.0 |
| 17 | 95.0 | 99.0 | 4.6 |
| 18 | 83.0 | 96.0 | 6.9 |
| 19 | 99.0 | 99.6 | 5.3 |
| 19a | 99.9 | 99.9 | 2.4 |
| 20 | 99.9 | 99.9 | 4.4 |
| 33 | 97.0 | 97.0 | 5.9 |
| 33a | 99.7 | 99.9 | 3.3 |
| 33b | 99.9 | 99.9 | |
| 33c | 99.9 | 99.9 | 1.9 |
| 33d | 99.9 | 99.9 | 2.2 |
| 33e | 99.8 | 99.9 | 3.1 |
| 33f | 99.8 | 99.9 | 2.8 |
| Chloroquindiphosphat | 99.9 | 99.9 | 2.4 |

Wie eingangs erwähnt, sind Arzneimittel, enthaltend eine Verbindung der Formel I oder ein pharmazeutisch annehmbares Säureadditionssalz davon und ein therapeutisch inertes Excipiens. ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe zusammen mit einem der mehreren therapeutisch inerten Trägern in eine galenische Darreichungsform bringt.

Die Verbindungen der Formel I und pharmazeutisch annehmbare Säureadditionssalze davon können als Heilmittel, z.B. in Form von Arzneimitteln, Verwendung finden. Diese Arzneimittel können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Arzneimitteln können die Verbindungen der Formel I und pharmazeutisch annehmbare Säureaddiionssalze davon mit pharmazeutisch inerten, anorganischen oder organisch Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösung und Sirupen eignen sich Träger beispielsweise Wasser, Polyole, Saccharose, Invertzucker, Glukose und dergleichen. Für wässerige Injektionslösungen wasserlöslicher Säureadditionssalze von Verbindungen der Formel I sind Hilfsstoffe, wie Alkohole, Polyole, Glyzerin, pflanzliche Oele und dergleichen verwendbar aber in der Regel nicht notwendig. Für Suppositorien eignen sich als Träger beispielsweise natürlich oder gehärtete Oele, Wachse, Fette, halbflüssige oder flüssige Polyole und dergleichen.

Die Arzneimittel können daneben noch Konservierungsmittel, Lösungsvermittler, Stabilisierungsmittel, Netzmitte, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Die Dosierung kann innerhalb weiter Grenzen variieren und ist natürlich in jedem einzelnen Fall den individuellen Gegebenheiten anzupassen. Im allgemeinen dürfte eine Tagesdosis von etwa 1 mg bis 1000 mg angemessen sein.

In den nachfolgenden Beispielen, welche die vorliegende Erfindung illustrieren, deren Umfang jedoch in keiner Weise beschränken sollen, sind sämtliche Temperaturen in Celsius-Graden angegeben. Die 250 Mz-1H-NMR Spektren wurden bei Raumtemperatur aufgenommen; chemische Verbindungen δ (ppm) relativ zu δ (TMS) = 0.0 ppm.

Beispiel 1

(RS)-$N_2$-(7-Chlor-chinolin-4-yl)-$N_1$,$N_1$-dimethyl-propan-1,2-diamin

5 g 4,7-Dichlorchinolin, 6.5 ml 1-N,N-Dimethylamino-2-propylamin und 0.8 g Phenol werden während 6 Stunden bei 140°C zur Reaktion gebracht. Nach Abkühlen fügt man 40 ml Wasser zu, stellt mittels konzentrierter Natronlauge auf pH 12 und extrahiert dreimal mit je 150 ml Ethylacetat. Der nach Abdampfen des Lösungsmittels verbleibende Rückstand wird 1x aus Isopropanol und 1x aus Acetonitril umkristallisiert. Man erhält 3.48 g farblose Kristalle, F: 168 - 170°C.

$^1$H-NMR in $CDCl_3$, δ (ppm): 1.29 (d, J = 7 Hz, 3H), 2.24 (s, 6H), 2.36 (dd, J = 6Hz und 12.5 Hz, 1H), 2.59 (dd, J = 9 Hz und 12.5 Hz, 1H), 3.62 (m, 1H), 5.90 (breit, 1H), 6.44 (d, J = 6 Hz, 1H), 7.34 (dd, J = 3 Hz und 9 Hz, 1H), 7.74 (d, J = 9 Hz, 1H), 7.95 (d, J = 3 Hz, 1 H), 8.53 (d, J = 6 Hz, 1H).

In analoger Weise lassen sich die folgenden Verbindungen herstellen:

Beispiel 2

(RS)-$N_2$-(7-Chlor-chinolin-4-yl)-$N_1$,$N_1$-diethyl-propan-1,2-diamin

3.91 g Base aus 5 g 4,7-Dichlorchinolin und 6 .56 g 1-N,N-Diethylamino-2-propylamin; farblose Kristalle aus Acetonitril, F: 90 - 92°C.

$^1$H-NMR in $CDCl_3$, δ (ppm): 1.01 (t, J = 7 Hz, 6H), 1.30 (d, J = 6.5 Hz, 3H), 2.4 - 2.7 (m, 6H), 3.57 (m, 1H), 6.13 (br., 1H), 6.44 (d, J = 6 Hz, 1H), 7.36 (dd, J = 3 Hz und 9 Hz, 1H), 7.70 (d, J = 9 Hz, 1H), 7.95 (d, J = 3 Hz, 1 H), 8.53 (d, J = 6 Hz, 1H).

Durch Zugabe von HCl zu einer Lösung in Isopropanol erhält man das Dihydrochlorid; farblose Kristalle, F: 268 - 270°C.

Beispiel 3

(RS)-(7-Chlor-chinolin-4-yl)-(1-methyl-2-pyrrolidin-1-yl-ethyl)-amin

3.36 g Base aus 3.18 g 4,7-Dichlorchinolin und 4.12 g 2-(Pyrrolidin-1-yl-propyl)-amin; farblose Kristalle aus Acetonitril, F: 160 - 163°C.

$^1$H-NMR in $CDCl_3$, δ (ppm): 1.32 (t, J = 7 Hz, 3H), 1.75 (m, 4H), 2.55 (m, 5H), 2.83 (dd, J = 9 Hz und J = 12 Hz, 1H), 3.67 (m, 1H), 5.92 (br., 1H), 6.44 (d, J = 6 Hz, 1H), 7.35 (dd, J = 3 Hz und 9 Hz, 1H), 7.72 (d, J = 9 Hz, 1H), 7.95 (d, J = 3 Hz, 1 H), 8.52 (d, J = 6 Hz, 1H).

Durch Zutropfen einer Lösung der Base in isopropanolischer Salzsäure (0.35 N) zum 4-fachen Volumen Diethylether erhält man das Dihydrochlorid in Form farbloser Kristalle, die sich bei 143°C zersetzen.

Beispiel 4

(RS)-(7-Chlor-chinolin-4-yl)-(1-methyl-2-piperidin-1-yl-ethyl)-amin

3.62 g aus 5.22 g 4,7-Dichlorchinolin und 7.5 g 2-(Piperidin-1-yl-propyl)-amin; farblose Kristalle aus Ethanol, F: 168 - 171°C.

$^1$H-NMR in $CDCl_3$, δ (ppm): 1.30 (d, J = 6.5 Hz, 3H), 1.3 - 1.6 (m, 6H). 2.3 - 2.6 (m, 6H), 3.62 (m, 1H), 6.20 (breit, 1H), 6.44 (d, J = 6 Hz, 1H), 7.39 (dd, J = 3 Hz und 9 Hz, 1H), 7.75 (d, J = 9 Hz, 1H), 7.95 (d, J = 3 Hz, 1 H), 8.53 (d, J = 6 Hz, 1H).

Beispiel 5

$N_2$-(7-Chlor-chinolin-4-yl)-$N_1$,$N_1$-dimethyl-ethan-1,2-diamin

1.09 g aus 5.9 g 4,7-Dichlorchinolin und 5.3 g 2-Dimethylamino-ethylamin; farblose Kristalle aus Etylacetat, F: 122-124°C.

$^1$H-NMR in $CDCl_3$, δ (ppm): 2.30 (s, 6H), 2.68 (m, 2H), 3.28 (m, 2H), 5.91 (br.t, 1H), 6.37 (d, J = 5.5 Hz, 1H), 7.36 (dd, J = 2.5 Hz und 9 Hz, 1H), 7.71 (d, J = 9 Hz, 1H), 7.95 (d, J = 2.5 Hz, 1H), 8.53 (d, J = 5.5 Hz, 1H).

Beispiel 6

$N_2$-(7-Chlor-chinolin-4-yl)-$N_1$,$N_1$-diethyl-ethan-1,2-diamin

Aus 4,7-Dichlorchinolin und 2-(N,N-Dimethylamino)-ethylamin; farblose Kristalle aus Isopropanol, F: über 250°C.

$^1$H-NMR in DMSO-$d_6$/$D_2O$, δ (ppm): 1.28 (t, J = 7.5 Hz, 6H), 3.28 (q, J = 7.5 Hz, 4H), 3.49 (t, J = Hz, 2H), 4.00 (t, J = Hz, 2H),), 7.06 (d, J = 7 Hz, 1H), 7.75 (dd, J = 2 Hz und 9 Hz, 1H), 8.02 (d, J = 2 Hz, 1H), 8.59 (d, J = 7 Hz, 1 H), 8.65 (d, J = 9 Hz, 1H).

Beispiel 7

(7-Chlor-chinolin-4-yl)-(2-pyrrolidin-1-yl-ethyl)-amin

3.37 g Base aus 3.96 g 4,7-Dichlorchinolin und 5 ml 1-(2-Aminoethyl)-pyrrolidin; das Rohprodukt wird vor der Umkristallisation durch Filtration mit Ethylacetat über 300 g Aluminiumoxid (Aktivitätsstufe II) gereinigt. Aus 400 ml Eluat erhält man gelbliche Kristalle, die, aus 95 ml Acetenitril umkristallisiert, 3.37 g farbloses kristallines Produkt ergeben, F: 134 - 136°C.

$^1$H-NMR in $CDCl_3$, δ (ppm): 1.83 (m, 4H), 2.59 (m, 4H), 2.87 (dd, J = 6 Hz und 7 Hz, 2H), 3.33 (m, 2H), 5.92 (breit, 1H), 6.38 (d, J = 6 Hz, 1H), 7.36 (dd, J = 3 Hz und 9 Hz, 1H), 7.70 (d, J = 9 Hz, 1H), 7.95 (d, J = 3 Hz, 1 H), 8.53 (d, J = 6 Hz, 1H).

Durch Zugabe von HCl zu einer Lösung der Base in Isopropanol erhält man das Dihydrochlorid in Form farbloser Kristalle, F: 212 - 213°C.

Beispiel 8

(7-Chlor-chinolin-4-yl)-(2-piperidin-1-yl-ethyl)-amin

3.57 g Base aus 3.96 g 4,7-Dichlorchinolin und 4.8 g 1-(2-Aminoethyl)-piperidin (Reaktionsdauer, 5 Stunden bei 140°C); gelbliche Kristalle aus Acetonitril, F: 148 - 151°C.

$^1$H-NMR in $CDCl_3$; δ (ppm): 1.45 - 1.7 (m, 6H), 2.46 (m, 4H), 2.72 (t, J = 6 Hz, 2H), 3.29 (m, 2H), 6.12 (br., 1H), 6.36 (d, J = 6 Hz, 1H), 7.38 (dd, J = 2.5 Hz und 8.5 Hz, 1H), 7.67 (d, J = 8.5 Hz, 1H), 7.95 (d J = 2.5 Hz, 1H), 8.53

(d, J = 6 Hz).

Beispiel 9

(1RS,2RS)-$N_1$-(7-Chlor-chinolin-4-yl)-$N_2$,$N_2$-diethyl-cyclohexan-1,2-diamin

11.3 g Base aus 15.2 g 4,7-Dichlorchinolin und 13.4 g 2-(Diethylamino)-cyclohexylamin (Reaktionsdauer, 16 Stunden bei 140°C); farblose Kristalle aus Acetonitril, F: 110 - 114°C.

$^1$H-NMR in $CDCl_3$, δ (ppm): 0.98 (t, J = 7 Hz, 6H), 1.1 - 1.45 (m, 5H), 1.75 - 2.0 (m, 3H), 2.38 (m, 2H), 2.54 (m, 2H), 2.70 (m, 1H), 3.10 (m, 1H), 6.43 (d, J = 6 Hz, 1H), 6.55(br.s, 1H), 7.36 (dd, J = 2.5 Hz und 9 Hz, 1H), 7.71 (d, J = 9 Hz, 1H), 7.94 (d, J = 2.5 Hz, 1 H), 8.52 (d, J = 6 Hz, 1H).

Das Dihydrochlorid wird aus isopropanolischer Salzsäure erhalten; farblose Kristalle aus Acetonitril, Zers. bei 198°C.

Beispiel 10

(1RS,2RS)-(7-Chlor-chinolin-4-yl)-(2-pyrrolidin-1-yl-cyclohexyl)-amin

3.87 g aus 3.6 g 4,7-Dichlorchinolin und 3.06 g (2-Pyrrolidin-1-yl-cyclohexyl)-amin (Reaktionsdauer 24 Stunden bei 120°C); farblose Kristalle aus Acetonitril/Ethanol, F: 228 - 231°C.

$^1$H-NMR in DMSO-$d_6$, δ (ppm): 1.25 - 2.0 (m, 12H), 2.22 (m,1H), 3.11 (m, 1H), 3.40 (m, 1H), 3.98 (m, 1H), 4.47 (m, 1H), 7.16 (d, J = 7.5 Hz, 1H), 7.75 (dd, J = 2.5 Hz und 9.5 Hz, 1H), 8.12 (d, J = 2.5 Hz, 1H), 8.64 (d, J = 7.5 Hz, 1 H), 8.98 (d, J = 9.5 Hz, 1H), 9.65 (d, J = 9.5 Hz, 1H), 10.15 (br.s, 1H). 14.63 (br.s, 1H).

Beispiel 11

(1RS,2RS)-$N_1$-(7-Chlor-chinolin-4-yl)-$N_2$,$N_2$-diethyl-cyclopentan-1,2-diamin

8.16 g Base aus 16 g 4,7-Dichlorchinolin und 12.7 g 2-(Diethylamino)-cyclopentylamin (16 Stunden Reaktionsdauer bei 140°C). Mit isopropanolischer Salzsäure erhält man daraus 7.84 g Dihydrochlorid; farblose Kristalle aus Acetonitril/Ethanol, F: 186 - 190°C.

$^1$H-NMR in DMSO-$d_6$, δ (ppm): 1.45 (d, J = 6 Hz, 6H), 1.8 - 2.4 (m, 6H), 2.56 (m, 1H), 3.1 - 3.5 (m, 4H), 5.20 (m, 2H), 6.90 (d, J = 7.5 Hz, 1H), 7.45 (dd, J = 2.5 Hz und 9.5 Hz, 1H), 8.15 (d, J = 2.5 Hz, 1H), 8.24 (d, J = 7.5 Hz, 1 H), 9.41 (d, J = 9.5 Hz, 1H), 11.55 (br.s, 1H). 14.55 (br.s, 1H).

Beispiel 11a

(1RS,2RS)-$N_1$-(7-Chlor-chinolin-4-yl)-(2-pyrrolidin-1-yl-cyclopentyl)-amin

1.78 g Base aus 3.56 g 4,7-Dichlorchinolin und 2.79 g 2-(Pyrrolidin-1-yl)-cyclopentylamin.

| $C_{18}H_{22}ClN_3$(315.85): | | | | |
|---|---|---|---|---|
| ber.: | C 68.45%, | H 7.02%, | Cl 11.22%, | N 13.30% |
| gef.: | C 68.32%, | H 7.00%, | Cl 11.36%, | N 13.11% |

Mit isopropanolischer Salzsäure erhält man daraus 1.64 g Dihydrochlorid; farblose Kristalle aus Acetonitril/Ethanol, F: 173°C (Zers.).

1H-NMR in DMSO-d6, δ (ppm): 1.7 - 2.0 (m, 8H), 2.3 - 2.4 (m, 2H), 3.09 (m, 2H), 3.53 (m, 2H), 4.40 (m, 1H), 4.79 (m, 1H), 7.01 (d, 1 H, J = 7 Hz), 7.79 (dd, J = 2.5 Hz und 9 Hz, 1H), 8.11 (d, J = 2.5 Hz, 1H), 8.66 (d, J = 7 Hz, 1

H), 9.04 (d, J = 9Hz, 1H), 9.86 (d, J = 7.5 Hz, 1H), 11.75 (br.s, 1H). 14.55 (br.s, 1H).

Beispiel 12

(7-Chlor-chinolin-4-yl)-(1-ethyl-pyrrolidin-2-yl-methyl)-amin

9.64 g Base aus 8.5 g 4,7-Dichlorchinolin und 11 g 2-(Aminomethyl)-1-ethyl-pyrrolidin. Das rohe Reaktionsprodukt wird vor der Umkristallisation durch Chromatographie an 400 g Aluminiumoxid (Aktivitätsstufe II) gereinigt; als Eluens dient ein 1:1-Gemisch von Etylacetat und Hexan. Nach einem Vorlauf von 400 ml wird mit 1.5 l Eluens das Produkt eluiert, das, aus 120 ml Hexan umkristallisiert, 9.64 g farblose Kristalle ergibt; F: 85 - 86°C.

Durch Lösen in 15 ml Isopropanol, Zugabe von 20 ml 3.6 N isopropanolischer Salzsäure und Zutropfen zu 100 ml Diethylether erhält man 12 g farbloses kristallines Dihydrochlorid, F: 239 - 240°C.

$^1$H-NMR in DMSO-$d_6$, δ (ppm): 1.28 (t, J = 7.5 Hz, 3H), 1.95 (m, 3H), 2.27 (m,1H), 3.16 (m, 2H), 3.48 (m, 2H), 3.88 (m, 1H), 4.14 (m, 2H), 7.09 (d, J = 8 Hz, 1H), 7.78 (dd, J = 3 Hz und 9 Hz, 1H), 8.24 (d, J = 3 Hz, 1H), 8.69 (d, J = 8 Hz, 1 H), 8.94 (d, J = 9 Hz, 1H), 10.08 (t, J = 6 Hz, 1H), 11.20 (br.s, 1H). 14.80 (br.s, 1H).

Beispiel 12a

(7-Chlor-chinolin-4-yl)-(1-methyl-pyrrolidin-2-yl-methyl)-amin

Analog zu Beispiel 12 erhält man 60 mg Base aus 2.91 g 4,7-Dichlorchinolin und 1.68 g 2-(Aminomethyl)-1-methyl-pyrrolidin; farblose Kristalle aus Hexan, F: 114 - 116°C.

$^1$H-NMR in DMSO-$d_6$, δ (ppm): 1.5 1.7 (m, 3H), 1.80 - 2.0 (m, 1H), 2.17 (m,1H), 2.35 (s, 3H), 2.55 - 2.60 (m, 1H), 2.98 (m, 1H), 3.14 (m, 1H), 3.40 (m, 1H), 6.50 (d, J = 5 Hz, 1H), 7.21 (t, J= 6 Hz, 1H), 7.45 (dd, J = 2.5 Hz und 9.5 Hz, 1H), 7.79 (d, J = 2.5 Hz, 1H), 8.24 (d, J = 5 Hz, 1 H), 8.40 (d, J = 9.5 Hz, 1H).

Beispiel 13

(7-Chlor-chinolin-4-yl)-(1,1-dimethyl-2-pyrrolidin-1-yl-ethyl)-amin

4.95 g 4,7-Dichlorchinolin, 7.1 g 2-Methyl-2-(pyrrolidin-1-yl)-propylamin und 5 g Phenol werden während 16 Stunden bei 140°C zur Reaktion gebracht. Nach dem Abkühlen der Schmelze gibt man 25 ml Wasser zu, stellt mit wenig konzentrierter Salzsäure auf pH 1 und schüttelt dreimal mit je 50 ml Ethylacetat aus. Danach fügt man konzentrierte Natronlauge bis zu pH 12 zu und extrahiert das Produkt dreimal mit je 100 ml Ethylacetat. Das nach dem Abdampfen des Lösungsmittels erhaltene Rohprodukt wird durch Chromatographie an 400 g Aluminiumoxid (Aktivitätsstufe II) mit einem Gemisch von Ethylacetat und Hexan (1:4) gereinigt. Nach Umkristallisieren aus Hexan erhält man 2.37 g farblose Kristalle, F: 89 - 91°C.

$^1$H-NMR in $CDCl_3$, δ (ppm): 1.48 (s, 6H), 1.86 (m, 4H), 2.71(s, 2H), 2.75 (m, 4H), 6.60 (d, J = 6 Hz, 1H), 6.84 (br., 1H), 7.33 (dd, J = 3 Hz und 9 Hz, 1H), 7.62 (d, J = 9 Hz, 1H), 7.91 (d, J = 3 Hz, 1 H), 8.45 (d, J = 6 Hz, 1H).

Das Dihydrochlorid wird durch Zugabe von 3.3 N HCl in Dioxan zu einer Lösung der Base in wasserfreiem Dioxan erhalten; farblose Kristalle, F: 249 - 252°C.

Beispiel 14

(7-Chlor-chinolin-4-yl)-(1,1-dimethyl-2-piperidin-1-yl-ethyl)-amin

4.75 g 4,7-Dichlorchinolin, 7.5 g 2-Methyl-2-(piperidin-1-yl)-propyl-amin und 0.75 g Phenol werden während 16 Stunden bei 180°C zur Reaktion gebracht. Das wie in dem vorangehende Beispiel isolierte rohe Reaktionsprodukt wird vor der Umkristallisation durch Chromatographie an 200 g Aluminiumoxid (Aktivitätsstufe II) gereinigt; als Eluens dient ein 1:1-Gemisch von Toluol und Aceton. Nach einem Vorlauf, der nicht umgesetztes 4,7-Dichlorchinolin enthält, wird das Produkt eluiert, das, aus 5 ml Acetonitril umkristallisiert, 0.8 g farblose Kristalle ergibt, F: 143 - 147°C.

$^1$H-NMR in $CDCl_3$, δ (ppm): 1.46 (s, 6H), 1.50 (m, 2H), 1.65 (m, 4H), 2.51 (s, 2H), 2.65 (m, 4H), 6.62 (d, J = 6 Hz, 1H), 6.83 (br.s, 1H), 7.37 (dd, J = 3 Hz und 9 Hz, 1H), 7.71 (d, J = 9 Hz, 1H), 7.93 (d, J = 3 Hz, 1 H), 8.46 (d, J = 6

Hz, 1H).

Beispiel 15

$N_3$-(7-Chlor-chinolin-4-yl)-$N_1$,$N_1$-dimethyl-propan-1,3-diamin

Aus 4,7-Dichlorchinolin und 3-(N,N-Dimethylamino)-propylamin; farblose Kristalle aus Ethylacetat-Hexan, F: 111-113°C.

$^1$H-NMR in $CDCl_3$, δ (ppm): 1.86 (quintett, J = 6 Hz, 2H), 2.37 (s, 6H), 2.58 (m, 2H), 3.39 (m, 2H), 6.31 (d, J = 5.5 Hz, 1H), 7.32 (dd, J = 2 Hz und 9 Hz, 1H), 7.58 (d, J = 9 Hz, 1H), 7.92 (d, J = 2 Hz, 1H), 7.94 (br. s, 1H). 8.48 (d, J = 5.5 Hz).

Beispiel 16

$N_3$-(7-Chlor-chinolin-4-yl)-$N_1$,$N_1$-diethyl-propan-1,3-diamin

2.99 g Base aus 3.96 g 4,7-dichlorchinolin und 5.2 g 3-(N,N-Diethylamino)-propylamin (Reaktionsdauer 4 Stunden bei 140°C); farblose Kristalle aus Acetonitril, F: 77 - 78°C.

$^1$H-NMR in $CDCl_3$, δ (ppm): 1.10 (t, J = 7 Hz, 6H), 1.92 (quintett, J = 6 Hz, 2H), 2.65 (m, 6H), 3.38 (m, 2H), 6.29 (d, J = 5.5 Hz, 1H), 7.32 (dd, J = 2.5 Hz und 9 Hz, 1H), 7.68 (d, J = 9 Hz, 1H), 7.92 (d J = 2.5 Hz, 1H), 8.18 (br. s, 1H). 8.50 (d, J = 5.5 Hz).

Das Dihydrochlorid gewinnt man mit isopropanolischer Salzsäure; farblose Kristalle aus Isopropanol, Zers. ab 118°C,

Beispiel 17

(7-Chlor-chinolin-4-yl)-(3-pyrrolidin-1-yl-propyl)-amin

3.08 g aus 7.72 g 4,7-Dichlorchinolin und 5 g 3-(Pyrrolidin-1-yl)-propylamin (Reaktionsdauer 16 Stunden bei 140°C); die Base wird mit isopropanolischer Salzsäure in das Dihydrochlorid übergeführt, das, aus Acetonitril/Ethanol umkristallisiert, farblose Kristalle ergibt, F: 204 - 206°C.

$^1$H-NMR in DMSO-$d_6$; δ (ppm): 1.96 (m, 4H), 2.14 (m, 2H), 3.00 (m, 2H), 3.26 (m, 2H), 3.51 (m, 2H), 3.67 (m, 2H), 6.96 (d, J = 7 Hz, 1H), 7.78 (dd, J = 2.5 Hz und 9 Hz, 1H), 8.13 (d, J = 2.5 Hz, 1H), 8.59 (d, J = 7 Hz, 1 H), 8.81 (d, J = 9 Hz, 1H), 9.86 (d, J = 6 Hz, 1H), 11.13 (br.s, 1H). 14.55 (br.s, 1H).

Beispiel 18

(7-Chlor-chinolin-4-yl)-(3-piperidin-1-yl-propyl)-amin

2.73 g aus 2.83 g 3-(Piperidin-1-yl)-propylamin und 3.94 g 4,7-Dichlorchinolin (Reaktionsdauer, 16 Stunden bei 140°C); die Base (gelbliche Kristalle aus Acetonitril, F: 113 - 116°C) wird mit isopropanolischer Salzsäure in das Dihydochlorid übergeführt, das aus Acetonitril/Ethanol umkristallisiert farbloses Kristalle, F: 137 - 141°C ergibt.

$^1$H-NMR in DMSO-$d_6$; δ (ppm): 1.28 (m, 1H), 1.54 - 1.76 (m, 5H), 2.08 (m, 2H), 2.78 (m, 2H), 3.07 (m, 2H), 3.27 - 3.38 (m, 2H), 3.53 (m, 2H), 6.21 (d, J = 7 Hz, 1H), 7.55 (dd, J = 2.5 Hz und 9 Hz, 1H), 8.01 (d, J = 2.5 Hz, 1H), 8.43 (d, J = 7 Hz, 1 H), 8.73 (d, J = 9 Hz, 1H), 9.88 (d, J = 6 Hz, 1H), 10.67 (br.s, 1H). 14.63 (br.s, 1H).

Beispiel 19

(1-Benzyl-piperidin-4-yl)-(7-chlor-quinolin-4-yl)-amin

5.65 g aus 10 g 4,7-Dichlorchinolin und 10.26 ml 4-Amino-1-benzylpiperidin; farblose Kristalle aus Acetonitril, F: 166 - 168°C. Hieraus erhält man aus isopropanolischer Salzsäure das Dihydrochlorid; farblose Kristalle aus Acetonitril/Ethanol, F über 250°C.

$^1H$-NMR in DMSO-$d_6$; δ (ppm): 2.1 - 2.4 (m, 4H), 3.04 (m, 2H), 3.44 (m, 2H), 4.08 (br.s, 1H), 4.30 (s, 2H), 7.04 (d, J = 7.5 Hz, 1H), 7.48 (m, 3H), 7.67 (m, 2H), 7.78 (dd, J = 2.5 Hz und 9 Hz, 1H), 8.12 (d, J = 2.5 Hz, 1H), 8.61 (d, J = 7.5 Hz, 1 H), 8.81 (d, J = 9.5 Hz, 1H), 9.30 (d, J = 7.5 Hz, 1H), 11.50 (br.s,1H).14.55(br.s,1H).

Beispiel 19a

(RS)-(7-chlor-chinolin-4-yl)-(1-methyl-piperidin-3-yl)-amin

4 g aus 11.29 g 4,7-Dichlorchinolin und 6.5 g 3-Amino-1-methyl-piperidin; farblose Kristalle aus Acetonitril, F: 149 - 150°C.

| $C_{15}H_{18}ClN_3$ (275.78): | | | | |
|---|---|---|---|---|
| ber.: | C 65.33%, | H 6.58%, | Cl 12.86%, | N 15.24% |
| gef.: | C 65.25%, | H 6.64%, | Cl 12.87%, | N 15.11% |

$^1H$-NMR in DMSO-$d_6$; δ (ppm): 1.3 - 1.95 (mehrere m, 6H), 2.02 (s, 3H), 2.67 (m, 1H), 2.92 (m, 1H), 3.67 (m, 1H), 6.54 (d, J = 6 Hz, 1H), 6.83 (d, J = 8 Hz, 1H), 7.44 (dd, J = 2.5 Hz und 9 Hz, 1H), 7.78 (d, J = 2.5 Hz, 1H), 8.32 (d, J = 9 Hz, 1 H), 8.39 (d, J = 6 Hz, 1H).

Beispiel 21

$N_1N_1$-Diethyl-$N_2$-(7-trifluormethyl-chinolin-4-yl)-ethan-1,2-diamin

5 g 4-Chlor-7-(trifluoromethyl)-chinolin und 10 g 2-N,N-Diethylamino-ethylamin werden in 150 ml Dimethylacetamid während 16 Stunden bei 120°C gerührt. Nach dem Abkühlen engt man im Wasserstrahlvakuum ein und nimmt den Rückstand mit 150 ml Wasser und etwas Salzsäure auf, so dass der pH 4.8 beträgt. Nach dreimaligem Extrahieren mit je 100 ml Ethylacetat stellt man mittels 1N Natronlauge auf pH 10.2 und extrahiert das basische Produkt mit 3x100 ml Ethylacetat. Nach dem Abdampfen des Lösungsmittels kristallisiert man aus Acetonitril um und erhält 1.96 farblose Kristalle, F: 122 - 124°C.

$^1H$-NMR in $CDCl_3$; δ (ppm): 1.08 (t, J = 7 Hz, 6H), 2.61 (q, J = 7 Hz, 4 H), 2.83 (t, J = 6 Hz, 2H), 3.27 (m, 2H), 6.23 (m, 1H), 6.45 (d, J = 6 Hz, 1H), 7.60 (dd, J = 3 Hz und 9 Hz, 1H), 7.85 (d, J = 9 Hz, 1H), 8.26 (m, 1H), 8.61 (d, J = 6 Hz, 1H).

In analoger Weise wurde hergestellt:

Beispiel 22

$N_1$,$N_1$-Dimethyl-$N_2$-(7-trifluormethyl-chinolin-4-yl)-ethan-1,2-diamin

Aus 4-Chlor-7-(trifluoromethyl)-chinolin und 2-N,N-Dimethylamino-ethylamin; farblose Kristalle aus Hexan, F: 110 - 113°C

$^1H$-NMR in $CDCl_3$; δ (ppm): 2.31 (s, 6H), 2.69 (m, 2H), 3.29 (m, 2H), 6.05 (m, 1H), 6.44 (d, J = 6 Hz, 1H), 7.51 (dd, J = 3 Hz und 9 Hz, 1H), 7.90 (d, J = 9 Hz, 1H), 8.26 (m, 1H), 8.61 (d, J = 6 Hz, 1H).

Beispiel 23

(RS)-(7-Chlor-chinolin-4-yl)-[2-(3-methyl-piperidin-1-yl)-ethyl]-amin

2.5 g Hydrochlorid des N-(2-Chlorethyl)-7-chlor-4-chinolinamin werden in 25 ml 3-Methylpiperidin während 24 Stunden in einem geschlossenen Rohr bei 100 °C gehalten. Danach gibt man 100 ml Wasser zu, stellt mittels konzentrierter Natronlauge auf pH 12 und extrahiert dreimal mit je 50 ml Ethylacetat. Die vereinigten Extrakte werden mit wenig Wasser gewaschen und eingeengt. Der Rückstand wird aus 80 ml Aceton umkristallisiert. Man erhält 0.88g farblose Kristalle, F: 148 - 151°C.

$^1$H-NMR in $CDCl_3$; δ (ppm): 0.90 (d, J = 6 Hz, 3H), 0.98 (m, 1H), 1.6 - 1.75 (m, 5H), 2.04 (dt, J = 3.5 Hz und 7 Hz, 1H), 2.75 (m, 2H), 2.84 (m,2H), 3.31 (m, 2H), 6.20 (breit, 1H), 6.37 (d, J = 6 Hz, 1H), 7.38 (dd, J = 3 Hz und 9 Hz, 1H), 7.70 (d, J = 9 Hz, 1H), 7.96 (d, J = 3 Hz, 1 H), 8.53 (d, J = 6 Hz, 1H).

In analoger Weise lassen sich unter Verwendung der geeigneten Amine die folgenden Verbindungen herstellen:

Beispiel 24

(7-Chlor-chinolin-4-yl)-[2-(4-methyl-piperidin-1-yl)-ethyl]-amin

2.72 g aus 2.5 g Hydrochlorid des N(2-Chlorethyl)-7-chlor-4-chinolinamin; farblose Kristalle aus Acetonitril, F: 154 - 156°C.

$^1$H-NMR in $CDCl_3$; δ (ppm): 0.96 (d, J = 6 Hz, 3H), 1.28 (m, 2H), 1.43 (m, 1H), 1.68 (m, 1H), 2.06 (m, 2H), 2.73 (m, 2H), 2.90 (m,2H), 3.29 (m, 2H), 6.10 (breit, 1H), 6.36 (d, J = 6 Hz, 1H), 7.36 (dd, J = 3 Hz und 9 Hz, 1H), 7.68 (d, J = 9 Hz, 1H), 7.95 (d, J = 3 Hz, 1 H), 8.52 (d, J = 6 Hz, 1H).

Beispiel 25

(7-Chlor-chinolin-4-yl)-[2-(3,3-dimethyl-piperidin-1-yl)-ethyl]-amin

2.1 g aus 2.5 g Hydrochlorid des N-(2-Chlorethyl)-7-chlor-4-chinolinamin ; farblose Kristalle aus Acetonitril, F: 151 - 153°C.

$^1$H-NMR in $CDCl_3$; δ (ppm): 0.98 (s, 6H), 1.29 (m, 2H), 1.66 (m, 1H), 2.12 (m, 2H), 2.45 (m, 2H), 2.69 (t, J = 6 Hz, 2H), 3.28 (m, 2H), 6.20 (breit, 1H), 6.36 (d, J = 6 Hz, 1H), 7.37 (dd, J = 3 Hz und 9 Hz, 1H), 7.68 (d, J = 9 Hz, 1H), 7.96 (d, J = 3 Hz, 1H), 8.52 (d, J = 6 Hz, 1H).

Beispiel 26

(RS)-(7-Chlor-chinolin-4-yl)-[2-(2-methyl-piperidin-1-yl)-ethyl]-amin

1.4 g aus 2.5 g Hydrochlorid des N-(2-Chlorethyl)-7-chlor-4-chinolinamin; farblose Kristalle aus Acetonitril, F: 133 - 134°C.

$^1$H-NMR in $CDCl_3$; δ (ppm): 1.12 (d, J = 6.5 Hz,6H), 1.30-1.75 (m, 6H), 2.17 (dt, J = 4 Hz und 10 Hz, 2H), 2.45 - 2.55 (m, 2H), 2.86 (tm, 1H), 3.1 - 3.3 (m, 3H), 6.13 (breit, 1H), 6.36 (d, J = 6 Hz, 1H), 7.38 (dd, J = 3 Hz und 9 Hz, 1H), 7.67 (d, J = 9 Hz, 1H), 7.95 (d, J = 3 Hz, 1 H), 8.52 (d, J = 6 Hz, 1H).

Beispiel 27

(RS)-(7-Chlor-chinolin-4-yl)-[2-(2-ethyl-piperidin-1-yl)-ethyl]-amin

1.52 g aus 2.5 g Hydrochlorid des N-(2-Chlorethyl)-7-chlor-4-chinolinamin; farblose Kristalle aus Acetonitril, F: 115 - 117°C.

$^1$H-NMR in $CDCl_3$; δ (ppm): 0.93 (t, J = 7.5Hz, 3H), 1.3 - 1.75 (m, 8H), 2.21 (m, 2H), 2.39 (m, 2H), 2.57 (m, 1H), 2.85 (m, 1H), 3.16 (m, 1H), 3.26 (m, 2H), 5.17 (breit, 1H), 6.36 (d, J = 6 Hz, 1H), 7.37 (dd, J = 3 Hz und 9 Hz, 1H), 7.65 (d, J = 9 Hz, 1H), 7.95 (d, J = 3 Hz, 1 H), 8.52 (d, J = 6 Hz, 1H).

Beispiel 28

(2R,6S)-(7-Chlor-chinolin-4-yl)-[2-(2,6-dimethyl-piperidin-1-yl)-ethyl]-amin

0.4 g aus 2.5 g Hydrochlorid des N-(2-Chlorethyl)-7-chlor-4-chinolinamin; farblose Kristalle aus Acetonitril, F: 138 - 139°C.

$^1$H-NMR in $CDCl_3$; δ (ppm): 1.14 (d, J = 6.5 Hz, 6H), 1.2 - 1.7 (m, 6H), 2.57 (m, 2H), 2.94 (t, J = 7 Hz, 2H), 3.31 (m, 2H), 5.85 (breit, 1H), 6.38 (d, J = 6 Hz, 1H), 7.37 (dd, J = 2.5 Hz und 9.5 Hz, 1H), 7.70 (d, J = 9.5 Hz, 1H), 7.95 (d, J = 2.5 Hz, 1 H), 8.53 (d, J = 6 Hz, 1H).

Beispiel 29

(7-Chlor-chinolin-4-yl)-[2-(3,5-dimethyl-piperidin-1-yl)-ethyl]-amin

0.67 g (Isomerengemisch cis:trans = 3:1) aus 2.5 g Hydrochlorid des N-(2-Chlorethyl)-7-chlor-4-chinolinamin; farblose Kristalle aus Acetonitril, F: 142 - 143°C.

$^1$H-NMR in $CDCl_3$; δ (ppm): 0.88 und 1.00(d, J = 6.5 Hz, 6H; Verhältnis 3 : 1), 1.3 - 2.0 (mehrere m, 4H), 2.2 - 2.84 (mehrere m, 6H), 3.29 (m, 2H), 6.12 und 6.20 (breit, 1H; Verhältnis 3:1), 6.37 (d, J = 6 Hz, 1H), 7.37 (dd, J = 3 Hz und 9 Hz, 1H), 7.66 (d, J = 9 Hz, 1H), 7.95 (d, J = 3 Hz, 1 H), 8.53 (d, J = 6 Hz, 1H).

Beispiel 30

(7-Chlor-chinolin-4-yl)-[2-(2,5-dimethyl-pyrrolidin-1-yl)-ethyl]-amin

0.35 g (Isomerengemisch cis:trans = 15 : 1) aus 1.3 g Hydrochlorid des N-(2-Chlorethyl)-7-chlor-4-chinolinamin; gelbliche Kristalle aus Aceton, F: 127 - 130°C.

$^1$H-NMR in DMSO-$D_6$; δ (ppm): 0.94 und 1.06(d, J = 6.5 Hz, 6H; Verhältnis 15 : 1), 1.29 (m, 2H), 1.80 (m, 2H), 2.67 (m, 2H), 2.79 (m, 2H), 3.34 (m, 2H),), 6.47 (d, J = 6 Hz, 1H), 7.05 (br.t, 1H), 7.46 (dd, J = 3 Hz und 9 Hz, 1H), 7.78 (d, J = 3 Hz, 1H), 8.19 (d, J = 9 Hz, 1 H), 8.40 (d, J = 6 Hz, 1H).

Beispiel 31

(7-Chlor-chinolin-4-yl)-[2-octahydro-indol-1-yl)-ethyl]-amin

0.3 g aus 2.2 g Hydrochlorid des N-(2-Chlorethyl)-7-chlor-4-chinolinamin; farblose Kristalle aus Ethylacetat, F: 115 - 117°C.

$^1$H-NMR in DMSO-$d_6$; δ (ppm): 1.1 - 2.0 (m, 11H), 2.15 - 2.45 (m, 2H), 2.95 (m, 1H), 3.18 (m, 1H), 3.36 (m, 4H), 6.48 (d, J = 6 Hz, 1H), 7.22 (t, J = 6 Hz, 1H), 7.45 (dd, J = 3 Hz und 9 Hz, 1H), 7.91 (d, J = 3 Hz, 1H), 8.19 (d, J = 9 Hz, 1 H), 8.40 (d, J = 6 Hz, 1H).

Beispiel 32

[2-(3-Azabicyclo[3.2.2]nonan-3-yl)-ethyl]-(7-chlor-chinolin-4-yl)-amin

0.45 g aus 1.48 g Hydrochlorid des N-(2-Chlorethyl)-7-chlor-4-chinolinamin; gelbliche Kristalle aus Ethanol, F: 205 - 207°C.

$^1$H-NMR in $CDCl_3$; δ (ppm): 1.60 - 1.82 (m, 8H), 1.95 (br.s, 2H), 2.66 (d, J = 4.5 Hz, 4H), 2.81 (m, 2H), 3.27 (m, 2H), 6.30 (breit, 1H), 6.36 (d, J = 6 Hz, 1H), 7.39 (dd, J = 3 Hz und 9 Hz, 1H), 7.69 (d, J = 9 Hz, 1H), 7.95 (d, J = 3 Hz, 1 H), 8.53 (d, J = 6 Hz, 1H).

Beispiel 33

$N_1$-Allyl-$N_2$-(7-chlor-chinolin-4-yl)-$N_1$-methyl-ethan-1,2-diamin

0.56 g aus 2.5 g Hydrochlorid des N-(2-Chlorethyl)-7-chlor-4-chinolinamin; farblose Kristalle aus Acetonitril, F: 91 - 93°C.

$^1$H-NMR in DMSO-$d_6$; δ (ppm): 2.24 (s,3H),), 2.62 (t, J = 7 Hz, 2H), 3.04 (d, J = 7 Hz, 2H), 3.37 (m, 2H), 5.1 - 5.2 (m, 2H), 5.84 (m, 1H), 6.49 (d, J = 6 Hz, 1H), 7.21 (t, J = 6 Hz, 1H), 7.45 (dd, J = 3 Hz und 9 Hz, 1H), 7.78 (d, J = 3 Hz, 1H), 8.22 (d, J = 9 Hz, 1 H), 8.40 (d, J = 6 Hz, 1H).

Beispiel 33a

(R,S)-(7-Chlor-chinolin-4-yl)-(1-methyl-pyrrolidin-3-yl)-amin

0.42 g Dihydrobromid des (R,S)-(7-Chlor-chinolin-4-yl)-(pyrrolidin-3-yl)-amins wird in 2 ml Ameisensäure und 2 ml 37% Formalin-Lösung über Nacht unter Rückfluss gekocht. Danach gibt man 10 ml Wasser zu, stellt mit konc. Salzsäure auf pH 2 und zieht dreimal mit je 20 ml Ethylacetat aus. Man stellt danach den pH der wässrigen Phase durch Zugabe von konc. Natronlauge auf 12 und extrahiert dreimal mit je 20 ml Ethylacetat. Nach dem Abdampfen des Lösungsmittels kristallisiert man aus ter.-Butyl-methyl-ether und Aceton um und erhält 20 mg farblose Kristalle, F: 150 - 152°C.

$^1$H-NMR in DMSO-$d_6$; δ (ppm): 1.75 - 2 (m, 1H), 2.29 (s,3H),), 2.3 - 2.8 (mehrere m, 5H), 4.13 (m, 1H), 6.47 (d, J = 6 Hz, 1H), 7.29 (d, J = 6.5 Hz, 1H), 7.45 (dd, J = 3 Hz und 9 Hz, 1H), 7.78 (d, J = 3 Hz, 1H), 8.39 (d, J = 6 Hz, 1 H), 8.40 (d, J = 9 Hz, 1H).

Beispiel 33b

$N_1$-(7-chlor-chinolin-4-yl)-2,$N_2$,$N_2$-trimethyl-propan-1,2-diamin

30 g $N_1$-(7-chlor-chinolin-4-yl)-2-methyl-propan-1,2-diamin werden in 21.6 ml wässriger Formaldehyd-Lösung (37%) und 45.3 ml Ameisensäure während 3 Stunden unter Rückfluss gekocht. Danach dampft man im Vakuum zur Trockne, nimmt mit 100 ml Wasser auf und stellt mittels konc. Kalilauge auf pH 12. Das ausgefällte Produkt wird aus 250 ml Acetornitril umkristallisiert und ergibt 25.1 g, F 114 - 116°C.

| $C_{11}H_{20}ClN_3$(277.80): | | | | |
|---|---|---|---|---|
| ber.: | C 64.85%, | H 7.26%, | Cl 12.76%, | N 15.13% |
| gef.: | C 64.64%, | H 7.18%, | Cl 12.79%, | N 15.07% |

$^1$H-NMR in DMSO-$d_6$; δ (ppm): 1.09 (s, 6H), 2.22 (s, 6H),), 3.18 (d, J = 5.5 Hz, 2H), 6.46 (t, J = 5.5 Hz, 1H), 6.51 (d, J = 6 Hz, 1H), 7.48 (dd, J = 2 Hz und 9.5 Hz, 1H), 7.82 (d, J = 2.5 Hz, 1H), 8.07 (d, J = 9.5 Hz, 1 H), 8.42 (d, J = 6 Hz, 1H).

Beispiel 33c

(S)-$N_2$-(7-Chlor-chinolin-4-yl)-$N_1$,$N_1$-dimethyl-propan-1,2-diamin

19 g (S)-2-Amino-1-chlor-N-(7-chlor-chinolin-4-yl)-propan Hydrochlorid werden in 190 ml Dimethylaminlösung (33% in Ethanol) gelöst und im Autoklaven während 15 Std. bei 110°C gehalten. Nach Abkühlen und Eindampfen wird der Rückstand in 200 ml Dichlormethan gelöst und mit 200 ml 1N NaOH extrahiert. Die basische Wasserphase wird mit 200 ml Dichlormethan nachextrahiert. Die Dichlormethanphasen werden getrocknet und eingedampft. Das Rohprodukt wird an neutralem Aluminiumoxid (Akt. II) in Hexan/Essigester 2:1, dann in Essigester allein chromatographiert. Das

resultierende Öl wird mit 100 ml Diethylether verrührt und nach erfolgter Kristallisation noch mit 50 ml Hexan verdünnt. Man erhält 13.5 g (79%) weisse Kristalle; Smp. 110°C, $[\alpha]_D$ = +115° (c = 1.0, MeOH).
Zur Herstellung des Dihydrochlorids werden 19.8 g freie Base in 200 ml Aceton gelöst, in Eis gekühlt und dann mit 75 ml 2N HCl versetzt und 10 Min. gerührt. Nach Eindampfen wird noch mit 200 ml Toluol versetzt und wieder eingedamft. Das resultierende Öl wird unter Erwärmen in 200 ml Ethanol gelöst, filtriert und nach erfolgter Kristallisation mit 50 ml Diethylether verdünnt. Man erhält 23.7 g (94%) weisse Kristalle; F: >260°C, $[\alpha]_D$ = +136.5° (c = 1.0, MeOH).

$^1$H-NMR (250 MHz) in $d_6$-DMSO, Signale bei δ (ppm): 1.31 (d, J = 6.3, 3H), 2.81 (s, 6H), 3.39 (dd, 1H), 4.00 (dd, 1H), 4.73 (m, 1H), 7.08 (d, J = 7, 1H), 7.75 (dd, $J_1$ = 9, $J_2$ = 2, 1H), 8.15 (d, J = 2, 1H), 8.65 (d, J = 7, 1H), 9.06 (d, J = 9, 1H), 9.67 (d, J = 9, 1H), 10.5 (br, 1H), 15.0 (br, 1H)

Beispiel 33d

(R)-$N_2$-(7-Chlor-chinolin-4-yl)-$N_1$,$N_1$-dimethyl-propan-1,2-diamin

Analog zu Beispiel 33c lässt sich ausgehend vom (R)-2-Amino-1-chlor-N-(7-chlor-chinolin-4-yl)-propan Hydrochlorid das Hydrochlorid des (R)-$N_1$-(7-Chlor-chinolin-4-yl)-$N_2$,$N_2$ -dimethyl-propan-1,2-diamins herstellen; weisse Kristalle, F >260°C, $[\alpha]_D$ = -132° (c = 1.0, MeOH).

Beispiel 33e

(S)-$N_2$-(7-Chlor-chinolin-4-yl)-$N_1$,$N_1$-diethyl-propan-1,2-diamin

15 g (S)-2-Amino-1-chlor-N-(7-chlor-chinolin-4-yl)-propan Hydrochlorid werden in 100 ml Methanol und 50 ml Diethylamin gelöst und im Autoklaven während 15 Std. bei 125°C gehalten. Nach Abkühlen und Eindampfen wird der Rückstand in 200 ml Dichlormethan gelöst und mit 200 ml 1N NaOH extrahiert. Die basische Wasserphase wird mit 200 ml Dichlormethan nachextrahiert. Die Dichlormethanphasen werden getrocknet und eingedampft. Das Rohprodukt wird an neutralem Aluminiumoxid (Akt. II) in Hexan/Essigester 2:1 chromatographiert. Das resultierende Öl wird in 40 ml Diethylether gelöst und langsam mit 200 ml Hexan verdünnt. Man erhält 10.3 g (69%) weisse Kristalle; F: 89°C, $[\alpha]_D$ = +122.6° (c = 1.0, MeOH).
Zur Herstellung des Dihydrochlorids werden 18.1 g freie Base in 190 ml Aceton gelöst, in Eis gekühlt und dann mit 62.5 ml 2N HCl versetzt und 10 min. gerührt. Nach Eindampfen wird noch mit 200 ml Toluol versetzt und wieder eingedamft. Das resultierende Öl wird unter Erwärmen in 200 ml Ethanol gelöst, filtriert und auf 100 ml eingeengt und zur Kristallisation mit 2x50 ml Diethylether verdünnt. Man erhält 21.8 g (96%) weisse Kristalle; F: 165-170°C, $[\alpha]_D$ = +150.6° (c = 1.0, MeOH).

$^1$H-NMR (250 MHz) in $d_6$-DMSO, Signale bei δ (ppm): 1.26 (t, J = 7, 6H), 1.36 (d, J = 6.3, 3H), 3.17 (q, J = 7, 4H), 3.35 (dd, 1H), 4.01 (dd, 1H), 4.72 (m, 1H), 7.10 (d, J = 7, 1H), 7.75 (dd, $J_1$ = 9, $J_2$ = 2, 1H), 8.17 (d, J = 2, 1H), 8.65 (d, J = 7, 1H), 9.08 (d, J = 9, 1H), 9.81 (d, J = 9, 1H), 10.5 (br, 1H), 15.0 (br, 1H)

Beispiel 33f

(R)-($N_2$-(7-Chlor-chinolin-4-yl)-$N_1$,$N_1$-diethyl-propan-1,2-diamin

Analog zu Beispiel 33e lässt sich ausgehend vom Hydrochlorid des (R)-2-Amino-1-chlor-N-(7-chlor-chinolin-4-yl)-propans das Hydrochlorid des (R)-$N_1$-(7-Chlor-chinolin-4-yl)-$N_2$,$N_2$ -dimethyl-propan-1,2-diamins herstellen; weisse Kristalle, F: 165 - 170°C, $[\alpha]_D$ = -140.3° (c = 1.0, MeOH).

Beispiel 33g

(S)-$N_1$-(7-Chlor-chinolin-4-yl)-$N_2$,$N_2$-dimethyl-propan-1,2-diamin

1.2 g (S)-$N_2$,$N_2$-Dimethyl-1,2-propandiamin und 2 g 4,7-Dichlorchinolin werden in 4 ml 1-Methyl-2-pyrrolidon im Autoklaven während 6.5 Std. bei 150°C gehalten. Nach Abkühlen wird das Reaktionsgemisch in 20 ml 3N HCl gegossen und mit 4x20 ml Dichlormethan extrahiert. Die saure wässrige Phase wurde mit 28% NaOH basisch gestellt und dann mit 3x20 ml Dichlormethan extrahiert. Das Rohprodukt wird an neutralem Aluminiumoxid (Akt. II) in Hexan/Essigester 10:3 chromatographiert. Man erhält 1.25 g gelbes Öl. Dieses wird in 10 ml Ethanol gelöst und mit ethanolischer HCl-Lösung ins Dihydrochlorid überführt. Man erhält 450 mg weisse Kristalle;F: 165°C, $[\alpha]_D$ = +15.1° (c = 1.0, MeOH).

$^1H$-NMR (250 MHz) in $d_6$-DMSO, Signale bei δ (ppm): 1.34 (d ,J = 6.1, 3H), 2.79 (s, 6H), 3.80 (m, 2H), 4.01 (m, 1H), 7.16 (d, J = 7, 1H), 7.78 (dd, $J_1$ = 9, $J_2$ = 2, 1H), 8.17 (d, J = 2, 1H), 8.65 (d, J = 7, 1H), 8.93 (d, J = 9, 1H), 9.92 (br, 1H), 11.1 (br, 1H), 15.0 (br, 1H)

Beispiel 33h

(R)-$N_1$-(7-Chlor-chinolin-4-yl)-$N_2$,$N_2$-dimethyl-propan-1,2-diamin

Analog zu Beispiel 33g lässt sich ausgehend vom Hydrochlorid des (R)-$N_2$,$N_2$-Dimethyl-1,2-propandiamins das Hydrochlorid des (R)-$N_1$-(7-Chlorchinolin-4-yl)-$N_2$,$N_2$-dimethyl-propan-1,2-diamins herstellen; weisse Kristalle, F: 250°C, $[\alpha]_D$ = --9,4° (c = 1.0, MeOH).

| $C_{14}H_{19}Cl_3N_3$(336.69): | | | | |
|---|---|---|---|---|
| Ber.: | C 49.94%, | H 5.99%, | Cl 31.59%, | N 12.48% |
| Gef.: | C 49.69%, | H 6.18%, | Cl 31.85%, | N 12.28% |

**Herstellung der Zwischenprodukte**

Beispiel 34

(7-Chlor-chinolin-4-yl)-(2-chlor-ethyl)-amin

Zu einer Suspension von 12.3 g 2-(7-Chlor-chinolin-4-amino)-ethanol [hergestellt nach R.C.Elderfield et al., J.Am.Chem.Soc. 68, 1250 (1946)] tropft man unter Rühren 20 ml Thionylchlorid so langsam zu, dass die Temperatur ohne äussere Kühlung auf ca 30°C gehalten werden kann. Man rührt eine Stunde bei RT nach und dampft danach zur Trockne ein. Den Rückstand kristallisiert man aus 120 ml Eissessig um. Man erhält farblose Kristalle, die nach Suspendieren in 250 ml Toluol bei 50°C unter Wasserstrahlvakuum getrocknet werden. Man erhält 9.75 g farbloses kristallines Produkt, F: 239 - 240°C

$^1H$-NMR in $CDCl_3$; δ (ppm): 3.97 (s, 4H), 7.02 (d, J = 8 Hz, 1H), 7.80 (dd, J = 3 Hz und 9 Hz, 1H), 8.14 (d, J = 3 Hz, 1H), 8.60 (d, J = 8 Hz, 1 H), 8.75 (d, J = 9 Hz, 1H), 9.85 (br.s, 1H), 14.63 (br.s, 1H).

Beispiel 35

4-Brom-7-chlor-chinolin

Bei 60° gibt man zu 150 g Phosphoroxybromid portionenweise 83,9 g 7-Chlor-4-hydroxychinolin und erwärmt danach während 6 Stunden auf 140°C. Nach Abkühlen versetzt man mit 3 l Eiswasser und extrahiert mit 2 x je 1.5 l Dichlormethan. Nach Abdampfen des Lösungsmittels kristallisiert man aus 1.3 l Hexan und erhält 34 g Produkt als farblose Kristalle, F: 102-103°C.

Beispiel 36

1-Methyl-2-pyrrolidin-1-yl-ethylamin

Zu 16.5 ml Pyrrolidin tropft man bei 5°C 15 ml wässrige Formaldehyd-Lösung (37%) innert einer Stunde zu. Danach gibt man 57.2 ml Nitroethan zu und rührt die Mischung über Nacht bei RT. Man fügt 150 ml Ethylacetat und 400 ml gesättigte Kochsalz-Lösung zu, trennt die organische Phase ab und destilliert im Wasserstrahlvakuum. Bei 85-87°C geht das Produkt, 1-(2-Nitropropyl)-pyrrolidin (18.5 g) über.

13.1 g dieses Zwischenproduktes wird sodann in 100 ml Methanol nach Zugabe von 0.65 g Raney-Nickel unter 100 bar und bei 80°C hydriert. Das Produkt, 1-Methyl-2-pyrrolidin-1-yl-ethylamin wird durch Destillation bei 49-52°C im Wasserstrahlvakuum erhalten (4.7 g; farblose Flüssigkeit).

In analoger Weise lassen sich unter Verwendung der entsprechenden Amine die folgenden Diamine herstellen:

1-Methyl-2-piperidin-1-yl-ethylamin;
N,N-Diethyl-propan-1,2-diamin;
N,N-Dimethyl-propan-1,2-diamin.

Beispiel 37

N,N-Diethyl-cyclohexan-1,2-diamin

30 g Cyclohexenoxid werden mit 44.8 g Diethylamin und 32.6 g Lithiumperchlorat in 300 ml Acetonitril über Nacht unter Rühren und Rückfluss zur Reaktion gebracht. Danach wird 200 ml Wasser zugegeben und das Produkt mit 3 x je 150 ml Ethylacetat extrahiert. Nach Abdampfen des Lösungsmittels hinterbleibt 30.4 g 2-(Diethylamino)-cyclohexanol. Dieses wird ohne weitere Reinigung in 150 ml Tetrahydrofuran gelöst. Nach Zugabe von 38.6 g Phthalimid und 68.7 g Triphenylphosphin tropft man sodann 45.9 g Azodicarbonsäure-diethylester bei einer Temperatur von 10-15°C zu und rührt die Mischung über Nacht bei RT. Danach dampft man ein, suspendiert den Rückstand in verdünnter Salzsäure (200 ml, pH 1) und wäscht mit 3 x 150 ml Ethylacetat. Durch Zugabe von konzentrierter Natronlauge stellt man sodann auf pH 12-13 und extrahiert das Zwischenprodukt mittels 3 x je 200 ml Ethylacetat. Nach Abdampfen des Lösungsmittels hinterbleibt das N,N-Diethyl-2-phthalimido-cyclohexyl-amin (36.8 g) in Form gelber Kristalle.

Diese werden über Nacht in 250 ml konzentrierter Salzsäure unter Rückfluss verseift. Nach dreimaligem Waschen mittels je 200 ml Dichlormethan stellt man durch Zugabe von konzentrierter Natronlauge auf pH 13-14 und extrahiert mittels 3 x je 200 ml Ethylacetat das Produkt, das man nach Abdampfen des Lösungsmittels als gelbliches Oel erhält (13.4 g), welches ohne weitere Reinigung weiterverwendet wird.

Unter Verwendung von Pyrrolidin anstelle des Diethylamins wird in analoger Weise das

2-Pyrrolidin-1-yl-cyclohexylamin

hergestellt.

In analoger Weise wird aus Cyclopentenoxid und Diethylamin das

N,N-Diethyl-cyclopentan-1,2-diamin

hergestellt, sowie aus Cyclopentenoxid und Pyrrolidin das

2-Pyrrolidin-1-yl-cyclopentylamin.

Beispiel 38

1,1-Dimethyl-2-pyrrolidin-1-yl-ethylamin

18 ml 2-Nitropropan und 16.5 ml Pyrrolidin werden in 60 ml Dioxan gelöst. Bei 5°C gibt man 15 ml wässrige Formaldehyd-Lösung (37%) und 8 ml 2%ige Natronlauge zu. Man erwärmt unter Rühren auf 90°C während 1 Stunde, kühlt und extrahiert danach mit 3 x je 150 ml Ethylacetat. Bei der Destillation im Wasserstrahlvakuum erhält man 19 g 1-(2-Nitro-2-methyl-propyl)-pyrrolidin.

Dieses Zwischenprodukt wird sodann in 100 ml Methanol nach Zugabe von 1.5 g Raney-Nickel unter 100 bar und bei 80°C hydriert. Das Produkt 1,1-Dimethyl-2-pyrrolidin-1-yl-ethylamin wird durch Destillation im Wasserstrahlvakuum bei 52-57°C erhalten (13.1 g; farblose Flüssigkeit).

In analoger Weise lässt sich unter Verwendung von Piperidin anstelle des Pyrrolidins das

1,1-Dimethyl-2-piperidin-1-yl-ethylamin

herstellen.

Beispiel 39

3-Piperidin-1-yl-propylamin

3.7 g Piperidin und 2.3 g Acrylnitril werden zusammengegeben und während 3 Stunden auf 100°C erhitzt. Nach dem Abkühlen nimmt man in 40 ml Isopropanol auf und fällt mittels 12.8 ml 3,6 N isopropanolischer Salzsäure das Hydrochlorid des 3-(1-Piperidino)-propionitrils aus (farblose Kristalle, F 186-190°C, 6,68 g).

Dieses Zwischenprodukt wird in 31 ml 25%ige HCl und 31 ml Eisessig in Gegenwart von 0.13 g Platindioxid bei RT unter 10 bar hydriert. Nach Eindampfen der Hydrierlösung nimmt man 50 ml Wasser auf, stellt mittels konzentrierter Natronlange auf pH 13 und extrahiert mit 3 x je 150 ml Ethylacetat. Durch Destillation erhält man das 3-Piperidin-1-yl-propylamin als farbloses Oel, Kp. 45-50°C 10.1 mm Hg.

Beispiel 40

(R,S)-(7-Chlor-chinolin-4-yl)-(pyrrolidin-3-yl)-amin

9.85 g 3-Aminopyrrolidin wird bei 0°C in 90 ml Dichlormethan, 17.7 ml N,N-Dimethylformamid und 25.5 ml Triethylamin mit 16 g Benzoylchlorid benzoyliert. Nach Erwärmen auf Raumtemperatur filtriert man und dampft zur Trockne. Man nimmt mit 100 ml Wasser auf (pH 8), filtriert und dampft wiederum zur Trockne. Den Rückstand digeriert man nacheinander mit je 90 ml Toluol, Ethylacetat und Dichlormethan, wobei die Lösung jeweils vom zunächst öligen, später kristallinen Rückstand dekantiert wird. Die organischen Lösungen werden vereinigt und eingedampft. Das erhaltene dunkelgefärbte Öl (2.7 g) wird sodann mit 2.1 g 4,7-Dichlorchinolin und 2.1 g Phenol bei 140°C über Nacht zur Reaktion gebracht. Man nimmt das Reaktionsgemisch mit 50 ml Wasser auf, stellt durch Zugabe von konc. Salzsäure auf pH 2 und schüttelt dreimal mit je 50 ml Ethylacetat. Danach wird die wässrige Phase mittels konc. Natronlauge auf pH 12 gestellt und dreimal mit je 50 ml Ehtylacetat extrahiert. Nach Abdampfen des Lösungsmittels kristallisiert man aus 10 ml Ethylacetat/Toluol. Das erhaltene Produkt (0.6 g) wird sodann durch 12 stündiges Kochen in 10 ml 48% HBr zum Dihydrobromid des (R,S)-(7-Chlor-chinolin-4-yl)-(pyrrolidin-3-yl)-amin verseift.

Beispiel 41

$N_1$-(7-chlor-chinolin-4-yl)-2-methyl-propan-1,2-diamin

52.8 g 4,7-Dichlorchinolin und 30.85 g 1,2-Diamino-2-methylpropan werden unter Argon in 530 ml N-Methyl-2-pyrrolidon während 5 Stunden bei 150°C verrührt. Danach dampft man das Lösungsmittel im Vakkum ab und nimmt den Rückstand in 1250 ml Wasser auf. Nachdem man mittels konc. Salzsäure auf pH 1 gestellt hat, schüttelt man einmal mit 200 ml, zweimal mit je 100 ml Ethylacetat aus und versetzt anschliessend mit ca 170 ml 30% Kalilauge bis zu einem pH von 12. Dabei fallen 88 g des $N_1$-(7-chlor-chinolin-4-yl)-2-methyl-propan-1,2-diamin in kristalliner Form aus; F 169 - 171°C.

Beispiel 42

(S)-2-Amino-1-chlor-N-(7-chlor-chinolin-4-yl)-propan

23 g L-Alaninol und 57.4 g 4,7-Dichlorchinolin werden in 100 ml 1-Methyl-2-pyrrolidon während 6 Std. bei 150°C gehalten. Nach Abkühlen wird das Reaktionsgemisch in 500 ml kalte 2 N HCl gegossen und mit 3 x 200 ml Dichlormethan extrahiert. Die saure wässrige Phase wird mit 28% NaOH basisch gestellt, wobei das Rohprodukt ausfällt. Nach 30 Min. Rühren im Eisbad wird abfiltriert und dann aus 300 ml 2-Propanol/ 150 ml Ethanol umkristallisiert. Man erhält 51.2 g (74%) (S)-2-Amino-N-(7-chlor-chinolin-4-yl)-1-propanol; weisse Kristalle, F: 225°C, $[\alpha]_D$ = +35° (c = 1.0, MeOH).

$^1$H-NMR (250 MHz) in $d_6$-DMSO, Signale bei $\delta$ (ppm): 1.24 (d, J = 6.4, 3H), 3.45 (m, 1H), 3.58 (m, 1H), 3.73 (m, 1H), 4.87 (t, J = 5.6, 1H), 6.53 (d, J = 7, 1H), 6.84 (d, J = 7.7, 1H), 7.44 (dd, $J_1$ = 9, $J_2$ = 2, 1H), 7.78 (d, J = 2, 1H), 8.35 (d, J = 7, 1H), 8.39(d, J = 9, 1H)

27.3 g (S)-2-Amino-N-(7-chlor-chinolin-4-yl)-1-propanol werden in 270 ml Chloroform suspendiert. Dann wird unter Kühlen mit Eis eine Lösung von 72 ml Thionylchlorid in 70 ml Chloroform über 30 Min. dazugetropft, wobei die Temperatur 25°C nicht übersteigen soll. Anschliessend wird noch 30 Min. bei Raumtemperatur und 90 Min. bei 70°C gerührt. Das Reaktionsgemisch wird abgekühlt und eingedampft, dann mit Toluol versetzt und nochmals eingedampft. Der resultierende Schaum wird in 500 ml Ethanol unter Erwärmen gelöst, filtriert und auf ca. 300 ml eingeengt, worauf Kristallisation eintritt. Man erhält 31.6 g (94%) Hydrochlorid des (S)-2-Amino- 1-chlor-N-(7-chlor-chinolin-4-yl)-propans als weisse Kristalle; F: 210°C, $[\alpha]_D$ = +90° (c=1.0, MeOH).

In analoger Weise lässt sich das R-Enantiomere, (R)-2-Amino-1-chlor-N-(7-chlor-chinolin-4-yl)-propan als Hydrochlorid ausgehend von D-Alaninol herstellen; weisse Kristalle, F: 210°C, $[\alpha]_D$ = 88° (c=1.0, MeOH).

1H-NMR (250 MHz) in d6-DMSO, Signale bei d (ppm): 1.41 (d, J = 6.5, 3H), 3.88-4.06 (m, 2H), 4.48 (m, 1H), 7.03 (d, J = 7.3, 1H), 7.79 (dd, $J_1$ = 9, $J_2$ = 2, 1H), 8.15 (d, J = 2, 1H) 8.59 (d, J = 7.3, 1H), 8.89 (d, J = 9, 1H), 9.44 (d,

J = 8.4, 1H), 14.66 (s, 1H)

Beispiel 43

(S)-$N_2$,$N_2$-Dimethyl-1,2-propandiamin

7.6 g L-Alaninol werden in 20 ml Ameisensäure und 20 ml 37%iger Formaldehydlösung während 14 Std. bei 90°C gerührt. Nach Abkühlen wird das Reaktionsgemisch auf 100 g Eis und 60 ml 28% NaOH gegossen und mit 3x100 ml Dichlormethan extrahiert. Nach Trocknen und Eindampfen der Dichlormethanphasen wird das Rohprodukt bei Normaldruck destilliert. Man erhält 7.8 g (S)-2-Dimethylamino-1-propanol als farbloses Öl; Kp. 147°C, $[a]_D$ = + 2.7° (c = 1.0, MeOH).
7.3 g (S)-2-Dimethylamino-1-propanol, 37 g Triphenylphosphin und 20.8 g Phthalimid werden in 70 ml Tetrahydrofuran unter Argon suspendiert und im Eisbad gekühlt. Dann werden 24 ml Azodicarbonsäurediethylester über 30 Min. zugetropft und noch 5 Std. bei Raumtemperatur weitergerührt. Dann giesst man in 200 ml eiskalte 1N HCl und extrahiert mit 3x200 ml Essigester. Die saure wässrige Phase wird mit 28% NaOH basisch gestellt und dann mit 3x200 ml Dichlormethan extrahiert. Nach Trocknen und Eindampfen der Dichlormethanphasen werden die 12 g Rohprodukt (GC 78% + 16% Regioisomer) an Kieselgel in Essigester chromatographiert. Man erhält 6 g gelbes Öl, das sich verfestigt (Regioisomer nicht abgetrennt). Diese 6 g Produkt werden in 50 ml conc. HCl während 20 Std. am Rückfluss gekocht. Nach Abkühlen wird die ausgefallene Phthalsäure abfiltriert. Das Filtrat wird mit 2x50 ml Dichlormethan gewaschen und dann vorsichtig unter Kühlen im Eisbad mit festem KOH basich gestellt und schliesslich mit 3x80 ml Dichlormethan extrahiert. Nach Trocknen und Eindampfen der Dichlormethanphasen wird das Rohprodukt bei Normaldruck destilliert. Man erhält 1.2 g (S)-$N_2$,$N_2$-Dimethyl-1,2-propandiamin als farbloses Öl; Kp. 127°C.

$^1$H-NMR (250MHz) in $CDCl_3$, Signale bei d (ppm): 0.89 (d, J = 6.4, 3H), 1.37 (s, 2H), 2.22 (s, 6H), 2.48 - 2.67 (m, 3H)

In analoger Weise lässt sich ausgehend von D-Alaninol das R-Enantiomer (R)-$N_2$,$N_2$-Dimethyl-1,2-propandiamin, herstellen.

Beispiel A

(RS)-$N_2$-(7-Chlor-chinolin-4-yl)-$N_1$,$N_1$-dimethyl-propan-1,2-diamin kann als Wirkstoff nach an sich bekannten Methoden zu pharmazeutischen Präparaten nachfolgender Zusammensetzung formuliert werden:

| 1. Tabletten à 500 mg | |
|---|---|
| Wirkstoff | 500 mg |
| Lactose pulv. | 149 mg |
| Polyvinylpyrrolidon | 15 mg |
| Dioctylnatriumsulfosuccinat | 1 mg |
| Na-Carboxymethylstärke | 30 mg |
| Magnesiumstearat | 5 mg |
| | 700 mg |

| 2. Tabletten à 50 mg | |
|---|---|
| Wirkstoff | 50 mg |
| Lactose pulv. | 50 mg |

(fortgesetzt)

| 2. Tabletten à 50 mg | |
|---|---|
| Mikrokristalline Cellulose | 82 mg |
| Na-Carboxymethylstärke | 15 mg |
| | 200 mg |

| 3. Kapseln à 100 mg | |
|---|---|
| Wirkstoff | 100.0 mg |
| Lactose pulv. | 104,7 mg |
| Maisstärke | 70,0 mg |
| Hydroxypropylmethylcellulose | 10.0 mg |
| Dioctylnatriumsulfosuccinat | 0.3 mg |
| Talk | 12,0 mg |
| Magnesiumstearat | 3,0 mg |
| | 300,0 mg |

| 4. Suppositorien à 500 mg | |
|---|---|
| Wirkstoff | 500 mg |
| Suppositorienmasse | ad 2000 mg |

| 5. Weichgelatinekapseln à 100 mg | |
|---|---|
| Wirkstoff | 100 mg |
| Triglycerid mittelkettig | 300 mg |
| | 400 mg |

**Patentansprüche**

**1.** Verwendung von Aminochinolin-Derivaten der allgemeinen Formel

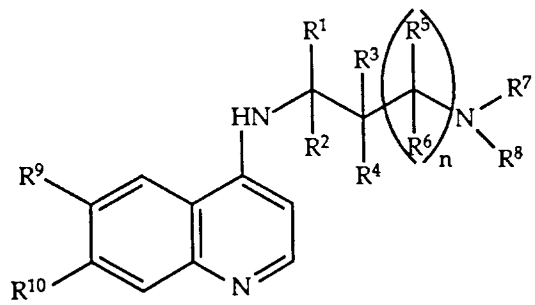

I

worin die Symbole $R^1$ bis $R^6$ Wasserstoff oder eines oder zwei davon ($C_1$-$C_4$)-Alkyl und die anderen Wasserstoff, $R^7$ und $R^8$ ($C_1$-$C_4$)-Alkyl, ($C_2$-$C_4$)-Alkenyl, Aryl-($C_1$-$C_4$)-Alkyl oder mit N zusammen Pyrrolidin oder Piperidin, auch substituiert durch ($C_1$-$C_4$)-Alkyl, Octahydroindol oder 3-Azabicyclo[3,2,2] nonan und n= 0 oder 1 oder
worin die Symbole $R^1$ und $R^3$ Tri- oder Tetramethylen, alle übrigen Substituenten bis $R^6$ Wasserstoff, n = 0 und $R^7$ und $R^8$ die obigen Bedeutungen besitzen oder
worin die Symbole $R^1$ und $R^7$ Methylen oder Dimethylen und n = 1,

$R^1$ und $R^7$ Di- oder Trimethylen und n = 0,
$R^3$ und $R^7$ Di- oder Trimethylen und n = 1
$R^3$ und $R^7$ Tri- oder Tetramethylen und n = 0
$R^5$ und $R^7$ Tri- oder Tetramethylen und n = 1
$R^1$ und $R^5$ Di- oder Trimethylen und n = 1, alle übrigen Substituenten Wasserstoff, ausser $R^8$, das ($C_1$-$C_4$)-Alkyl, $C_2$-$C_4$)-Alkenyl oder Aryl-($C_1$-$C_4$)-Alkyl bedeutet oder
worin die Symbole $R^3$ und $R^5$ Tri- oder Tetramethylen und n = 1, alle übrigen Substituenten bis $R^6$ Wasserstoff und $R^7$ und $R^8$ ($C_1$-$C_4$)-Alkyl, ($C_2$-$C_4$)-Alkenyl, Aryl-($C_1$-$C_4$)-Alkyl oder mit N zusammen Pyrrolidin oder Piperidin, auch substituiert durch ($C_1$-$C_4$)-Alkyl, $R^9$ Wasserstoff oder Halogen und $R^{10}$ Halogen oder Trifluormethyl bedeuten,

sowie pharmazeutisch annehmbare Salze von basischen Verbindungen der allgemeinen Formel I zur Herstellung von Arzneimitteln für die Bekämpfung von chloroquin-resistenten Malariaerregern.

2. Verwendung von Verbindungen der Formel I nach Anspruch 1, worin die Symbole $R^1$ bis $R^6$ Wasserstoff oder eines oder zwei davon ($C_1$-$C_4$)-Alkyl, und die anderen Wasserstoff, $R^7$ und $R^8$ ($C_1$-$C_4$)-Alkyl, ($C_2$-$C_4$)-Alkenyl, Aryl-($C_1$-$C_4$)-Alkyl oder mit N zusammen Pyrrolidin oder Piperidin, auch substituiert durch ($C_1$-$C_4$)-Alkyl, und n = 0 oder 1 bedeuten.

3. Verwendung von (S)-$N_2$-(7-Chlorchinolin-4-yl)-$N_1$,$N_1$-dimethyl-propan-1,2-diamin nach Anspruch 2.

4. Verwendung von (R)-$N_2$-(7-Chlor-chinolin-4-yl)-$N_1$,$N_1$-dimethyl-propan-1,2-diamin nach Anspruch 2.

5. Verwendung von $N_1$-(7-Chlor-chinolin-4-yl)-2,$N_2$,$N_2$-trimethyl-propan-1,2-diamin nach Anspruch 2.

6. Verwendung von $N_3$-(7-Chlor-chinolin-4-yl)-$N_1$,$N_1$-diethyl-propan-1,3-diamin nach Anspruch 2.

7. Verwendung von (RS)-(7-Chlor-chinolin-4-yl)-(1-methyl-piperidin-3-yl)amin nach Anspruch 2.

8. Verwendung von (RS)-(7-Chlor-chinolin-4-yl)-(1-methyl-pyrrolidin-3-yl)-amin nach Anspruch 2.

9. Verwendung von

(RS)-$N_2$-(7-Chlor-chinolin-4-yl)-$N_1$,$N_1$-dimethyl-propan-1,2-diamin,

(RS)-$N_2$-(7-Chlor-chinolin-4-yl)-$N_1$,$N_1$-diethyl-propan-1,2-diamin,

(S)-$N_2$-(7-Chlor-chinolin-4-yl)-$N_1$,$N_1$-diethylpropan-1,2-diamin,

(R)-$N_2$-(7-Chlor-chinolin-4-yl)-$N_1$,$N_1$-diethyl-propan-1,2-diamin,

(RS)-(7-Chlor-chinolin-4-yl)-(1-methyl-2-pyrrolidin-1-yl-ethyl)-amin,

$N_2$-(7-Chlor-chinolin-4-yl)-$N_1$,$N_1$-dimethyl-ethan-1,2-diamin,

$N_2$-(7-Chlor-chinolin-4-yl)-$N_1$,$N_1$-diethyl-ethan-1,2-diamin,

$N_3$-(7-Chlor-chinolin-4-yl)-$N_1$,$N_1$-dimethyl-propan-1,3-diamin,

(R)-$N_1$-(7-Chlor-chinolin-4-yl)-$N_2$,$N_2$-dimethyl-propan-1,2-diamin,

(S)-$N_1$-(7-Chlor-chinolin-4-yl)-$N_2$,$N_2$-dimethyl-propan-1,2-diamin und

(RS)-(7-Chlor-chinolin-4-yl)-(1-methyl-pyrrolidin-2-yl-methyl)-amin nach Anspruch 2.

**10.** Verbindungen der in Anspruch 1 definierten allgemeinen Formel I, worin $R^7$ und $R^8$ ($C_2$-$C_4$)-Alkenyl, Aryl-($C_1$-$C_4$)-Alkyl Octahydroindol oder 3-Azabicyclo[3,2,2]nonan bedeuten und alle übrigen Substituenten die Bedeutungen im Anspruch 1 besitzen.

**11.** Verfahren zur Herstellung von Verbindungen nach Anspruch 10, dadurch gekennzeichnet, dass man

a) Chinolinderivate der allgemeinen Formel worin $R^9$ und $R^{10}$ die in Anspruch 1 angegebenen Bedeutungen haben und R eine Abgangsgruppe bedeutet, mit Aminoverbindungen der allgemeinen Formel

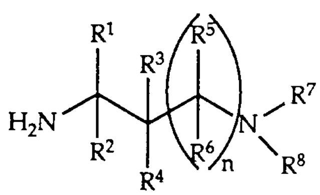

III

worin die Substituenten $R^1$ bis $R^8$ die in Anspruch 1 angegebenen Bedeutungen haben, umsetzt, oder

b) Alkylamino-Chinolinderivate der allgemeinen Formel

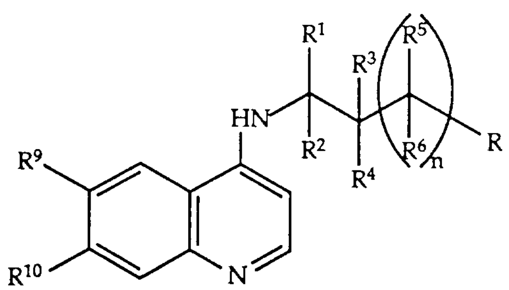

IV

worin $R^1$ bis $R^6$ und $R^9$ und $R^{10}$ die in Anspruch 1 angegebenen Bedeutungen haben und R eine Abgangsgruppe bedeutet, mit Aminen der Formel

$$HNR^7R^8 \quad V,$$

worin $R^7$ und $R^8$ die in Anspruch 1 angegebenen Bedeutungen haben, umsetzt, und

c) erwünschtenfalls eine basische Verbindung der Formel I mittels einer Säure in ein pharmazeutisch anwendbares Salz überführt.

**12.** Verbindungen nach Anspruch 10 zur Anwendung als therapeutische Wirkstoffe.

13. Verbindungen nach Anspruch 10 zur Anwendung als Wirkstoffe gegen sowohl chloroquin-resistente als auch chloroquin-sensitive Malariaerreger.

14. Verwendung von Verbindungen nach Anspruch 10 bei der Bekämpfung von Krankheiten bzw. bei der Verbesserung der Gesundheit.

15. Arzneimittel, enthaltend eine oder mehrere Verbindungen nach Anspruch 10 und ein therapeutisch inertes Excipiens.

16. Arzneimittel nach Anspruch 15 zur Behandlung oder Verhütung von Malaria.

## Claims

1. The use of aminoquinoline derivatives of the general formula

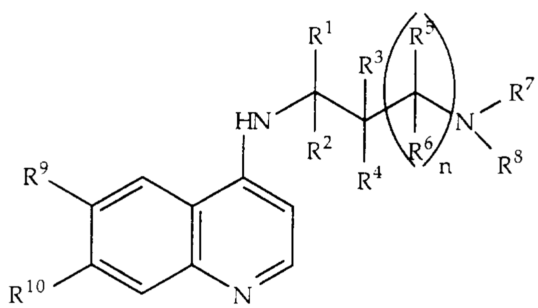

I

wherein the symbols $R^1$ to $R^6$ signify hydrogen or one or two thereof signifies (signify) $(C_1\text{-}C_4)$-alkyl and the others signify hydrogen, $R^7$ and $R^8$ signify $(C_1\text{-}C_4)$-alkyl, $(C_2\text{-}C_4)$-alkenyl, aryl-$(C_1\text{-}C_4)$-alkyl or together with the N atom signify pyrrolidine or piperidine, optionally substituted by $(C_1\text{-}C_4)$-alkyl, octahydroindole or 3-azabicyclo[3,2,2]nonane and n = 0 or 1 or

wherein the symbols $R^1$ and $R^3$ signify tri- or tetramethylene, all remaining symbols to $R^6$ signify hydrogen, n = 0 and $R^7$ and $R^8$ have the above significances or

wherein the symbols $R^1$ and $R^7$ signify methylene or dimethylene and n = 1,

$R^1$ and $R^7$ signify di- or trimethylene and n = 0,
$R^3$ and $R^7$ signify di- or trimethylene and n = 1
$R^3$ and $R^7$ signify tri- or tetramethylene and n = 0
$R^5$ and $R^7$ signify tri- or tetramethylene and n = 1
$R^1$ and $R^5$ signify di- or trimethylene and n = 1, all remaining substituents signify hydrogen, except $R^8$ which signifies $(C_1\text{-}C_4)$-alkyl, $(C_2\text{-}C_4)$-alkenyl or aryl-$(C_1\text{-}C_4)$-alkyl or

wherein the symbols $R^3$ and $R^5$ signify tri- or tetramethylene and n = 1, all remaining substituents to $R^6$ signify hydrogen and $R^7$ and $R^8$ signify $(C_1\text{-}C_4)$-alkyl, $(C_2\text{-}C_4)$-alkenyl, aryl-$(C_1\text{-}C_4)$-alkyl or together with the N atom signify pyrrolidine or piperidine, optionally substituted by $(C_1\text{-}C_4)$-alkyl,
$R^9$ signifies hydrogen or halogen and $R^{10}$ signifies halogen or trifluoromethyl,
as well as pharmaceutically acceptable salts of basic compounds of general formula I for the production of medicaments for the control of chloroquine-resistant malaria pathogens.

2. The use of compounds of formula I according to claim 1 in which the symbols $R^1$ to $R^6$ signify hydrogen or one or two thereof signifies (signify) $(C_1\text{-}C_4)$-alkyl and the others signify hydrogen, $R^7$ and $R^8$ signify $(C_1\text{-}C_4)$-alkyl, $(C_2\text{-}C_4)$-alkenyl, aryl-$(C_1\text{-}C_4)$-alkyl or together with the N atom signify pyrrolidine or piperidine, optionally substituted by $(C_1\text{-}C_4)$-alkyl, and n = 0 or 1.

3. The use of (S)-$N_2$-(7-chloro-quinolin-4-yl)-$N_1$,$N_1$-dimethyl-propane-1,2-diamine according to claim 2.

4. The use of (R)-$N_2$-(7-chloro-quinolin-4-yl)-$N_1$,$N_1$-dimethyl-propane-1,2-diamine according to claim 2.

**5.** The use of $N_1$-(7-chloro-quinolin-4-yl)-2,$N_2$,$N_2$-trimethyl-propane-1,2-diamine according to claim 2.

**6.** The use of $N_3$-(7-chloro-quinolin-4-yl)-$N_1$,$N_1$-diethyl-propane-1,3-diamine according to claim 2.

**7.** The use of (RS)-(7-chloro-quinoline-4-yl)-(1-methyl-piperidin-3-yl)amine according to claim 2.

**8.** The use of (RS)-(7-chloro-quinolin-4-yl)-(1-methyl-pyrrolidin-3-yl)-amine according to claim 2.

**9.** The use of:

(RS)-$N_2$-(7-chloro-quinolin-4-yl)-$N_1$,$N_1$-dimethyl-propane-1,2-diamine,

(RS)-$N_2$-(7-chloro-quinolin-4-yl)-$N_1$,$N_1$-diethyl-propane-1,2-diamine,

(S)-$N_2$-(7-chloro-quinolin-4-yl)-$N_1$,$N_1$-diethyl-propane-1,2-diamine,

(R)-$N_2$-(7-chloroquinolin-4-yl)-$N_1$,$N_1$-diethyl-propane-1,2-diamine,

(RS)-(7-chloro-quinolin-4-yl)-(1-methyl-2-pyrrolidin-1-yl-ethyl)amine,

$N_2$-(7-chloro-quinolin-4-yl)-$N_1$,$N_1$-dimethyl-ethane-1,2-diamine,

$N_2$-(7-chloro-quinolin-4-yl)-$N_1$,$N_1$-diethyl-ethane-1,2-diamine,

$N_3$-(7-chloro-quinolin-4-yl)-$N_1$,$N_1$-dimethyl-propane-1,3-diamine,

(R)-$N_1$-(7-chloro-quinolin-4-yl)-$N_2$,$N_2$-dimethyl-propane-1,2-diamine,

(S)-$N_1$-(7-chloro-quinolin-4-yl)-$N_2$,$N_2$-dimethyl-propane-1,2-diamine and

(RS)-(7-chloro-quinolin-4-yl)-(1-methyl-pyrrolidin-2-yl-methyl)amine
according to claim 2.

**10.** Compounds of general formula I defined in claim 1, wherein $R^7$ and $R^8$ signify ($C_2$-$C_4$)-alkenyl, aryl-($C_1$-$C_4$)-alkyl, octahydroindole or 3-azabicyclo[3,2,2]nonane and all remaining substituents have the significances in claim 1

**11.** A process for the manufacture of compounds according to claim 10, characterized by

a) reacting quinoline derivatives of the general formula

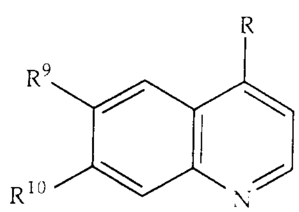

II

wherein $R^9$ and $R^{10}$ have the significances given in claim 1 and R signifies a leaving group, with amino compounds of the general formula

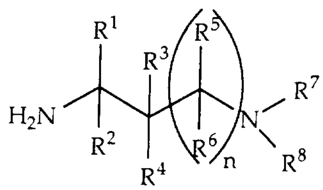

III

wherein the substituents $R^1$ to $R^8$ have the significances given in claim 1,

or

b) reacting alkylamino-quinoline derivatives of the general formula

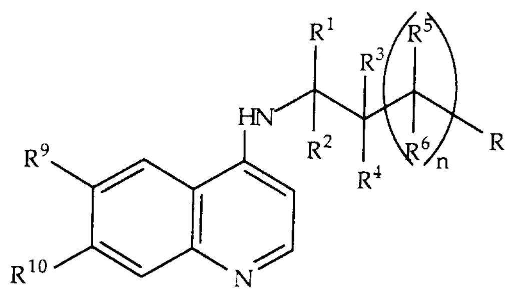

IV

wherein $R^1$ to $R^6$ and $R^9$ and $R^{10}$ have the significances given in claim 1 and R signifies a leaving group, with amines of the formula

$$HNR^7R^8 \qquad V$$

wherein $R^7$ and $R^8$ have the significances given in claim 1, and

c) if desired, converting a basic compound of formula I into a pharmaceutically usable salt by means of an acid.

**12.** Compounds according to claim 10 for use as therapeutically active substances.

**13.** Compounds according to claim 10 for use as active substances against not only chloroquine-resistant malaria pathogens, but also chloroquine-sensitive malaria pathogens.

**14.** The use of compounds according to claim 10 in the control of diseases or in the improvement of health.

**15.** A medicament containing one or more compounds according to claim 10 and a therapeutically inert excipient.

**16.** A medicament according to claim 15 for the treatment or prevention of malaria.

**Revendications**

**1.** Utilisation de dérivés d'aminoquinoléines, de formule générale

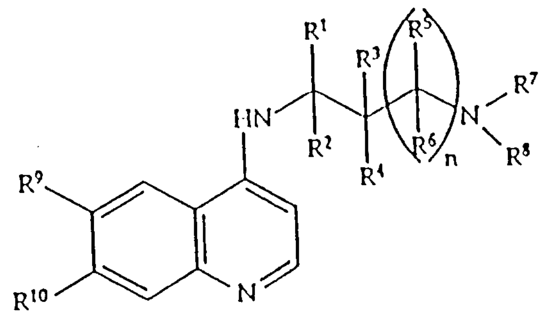

I

dans laquelle les symboles $R^1$ à $R^6$ représentent des atomes d'hydrogène, ou un ou deux d'entre eux représente(nt) un groupe alkyle en $C_1$-$C_4$ et les autres représentent des atomes d'hydrogène, $R^7$ et $R^8$ représentent un groupe alkyle en $C_1$-$C_4$, alcényle en $C_2$-$C_4$, aryl-alkyle-($C_1$-$C_4$), ou forment ensemble avec N un cycle pyrrolidine ou pipéridine également substitué par un groupe alkyle en $C_1$-$C_4$, octahydroindole ou 3-azabicyclo[3,2,2]nonane, et n= 0 ou 1, ou

dans laquelle les symboles $R^1$ et $R^3$ représentent le groupe tri- ou tétraméthylène, tous les autres substituants jusqu'à $R^6$ représentent des atomes d'hydrogène, n= 0 et $R^7$ et $R^8$ ont les significations données plus haut, ou

dans laquelle les symboles $R^1$ et $R^7$ représentent le groupe méthylène ou diméthylène et n= 1,

$R^1$ et $R^7$ représentent le groupe di- ou triméthylène et n= 0,
$R^3$ et $R^7$ représentent le groupe di- ou triméthylène et n= 1,
$R^3$ et $R^7$ représentent le groupe tri- ou tétraméthylène et n= 0,
$R^5$ et $R^7$ représentent le groupe tri- ou tétraméthylène et n= 1,
$R^1$ et $R^5$ représentent le groupe di- ou triméthylène et n= 1, tous les autres substituants représentent des atomes d'hydrogène, hormis $R^8$ qui représente un groupe alkyle en $C_1$-$C_4$, alcényle en $C_2$-$C_4$ ou aryl-alkyle($C_1$-$C_4$), ou

dans laquelle les symboles $R^3$ et $R^5$ représentent le groupe tri- ou tétraméthylène et n= 1, tous les autres substituants jusqu'à $R^6$ représentent des atomes d'hydrogène et $R^7$ et $R^8$ représentent un groupe alkyle en $C_1$-$C_4$, alcényle en $C_2$-$C_4$, aryl-alkyle($C_1$-$C_4$), ou forment ensemble avec N un cycle pyrrolidine ou pipéridine également substitué par un groupe alkyle en $C_1$-$C_4$, $R^9$ représente un atome d'hydrogène ou d'halogène et $R^{10}$ représente un atome d'halogène ou le groupe trifluorométhyle,

ainsi que les sels pharmaceutiquement acceptables des composés basiques de formule générale I, pour la fabrication de médicaments destinés à la lutte contre des agents pathogènes paludéens résistants à la chloroquine.

2. Utilisation des composés de formule I selon la revendication 1, dans lesquels les symboles $R^1$ à $R^6$ représentent des atomes d'hydrogène, ou un ou deux d'entre eux représente(nt) un groupe alkyle en $C_1$-$C_4$ et les autres représentent des atomes d'hydrogène, $R^7$ et $R^8$ représentent un groupe alkyle en $C_1$-$C_4$, alcényle en $C_2$-$C_4$, aryl-alkyle-($C_1$-$C_4$), ou forment ensemble avec N un cycle pyrrolidine ou pipéridine également substitué par un groupe alkyle en $C_1$-$C_4$, et n= 0 ou 1.

3. Utilisation de la (S)-$N_2$-(7-chloroquinolin-4-yl)-$N_1$,$N_1$-diméthylpropane-1,2-diamine selon la revendication 2.

4. Utilisation de la (R)-$N_2$-(7-chloroquinolin-4-yl)-$N_1$,$N_1$-diméthylpropane-1,2-diamine selon la revendication 2.

5. Utilisation de la $N_1$-(7-chloroquinolin-4-yl)-2,$N_2$,$N_2$-triméthylpropane-1,2-diamine selon la revendication 2.

6. Utilisation de la $N_3$-(7-chloroquinolin-4-yl)-($N_1$,$N_1$-diéthylpropane-1,3-diamine selon la revendication 2.

7. Utilisation de la (RS)-(7-chloroquinolin-4-yl)-(1-méthylpipéridine-3-yl)amine selon la revendication 2.

8. Utilisation de la (RS)-(7-chloroquinolin-4-yl)-(1-méthylpyrrolidine-3-yl)amine selon la revendication 2.

**9.** Utilisation de

la (RS)-$N_2$-(7-chloroquinolin-4-yl)-$N_1$,$N_1$-diméthylpropane-1,2-diamine,
la (RS)-$N_2$-(7-chloroquinolin-4-yl)-$N_1$,$N_1$-diéthylpropane-1,2-diamine,
la (S)-$N_2$-(7-chloroquinolin-4-yl)-$N_1$,$N_1$-diéthylpropane-1,2-diamine,
la (R)-$N_2$-(7-chloroquinolin-4-yl)-$N_1$,$N_1$-diéthylpropane-1,2-diamine,
la (RS)-(7-chloroquinolin-4-yl)-(1-méthyl-2-pyrrolidine-1-yl-éthyl)amine,
la $N_2$-(7-chloroquinolin-4-yl)-$N_1$,$N_1$-diéthyléthane-1,2-diamine,
la $N_2$-(7-chloroquinolin-4-yl)-$N_1$,$N_1$-diéthyléthane-1,2-diamine,
la $N_3$-(7-chloroquinolin-4-yl)-$N_1$,$N_1$-diméthylpropane-1,3-diamine,
la (R)-$N_1$-(7-chloroquinolin-4-yl)-$N_2$,$N_2$-diéthylpropane-1,2-diamine,
la (S)-$N_1$-(7-chloroquinolin-4-yl)-$N_2$,$N_2$-diéthylpropane-1,2-diamine et
la (RS)-(7-chloroquinolin-4-yl)-(1-méthylpyrrolidine-2-ylméthyl)amine
selon la revendication 2.

**10.** Composés de formule générale I définie dans la revendication 1, dans lesquels $R^7$ et $R^8$ représentent un groupe alcényle en $C_2$-$C_4$, aryl-alkyle($C_1$-$C_4$), octahydroindole ou 3-azabicyclo[3,2,2]nonane et tous les autres substituants ont les significations données dans la revendication 1.

**11.** Procédé pour la préparation des composés selon la revendication 10, caractérisé en ce que

a) on fait réagir des dérivés de quinoléine de formule générale

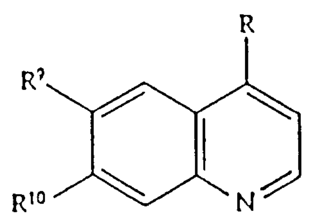

II

dans laquelle $R^9$ et $R^{10}$ ont les significations données dans la revendication 1 et R représente un groupe partant, avec des composés aminés de formule générale

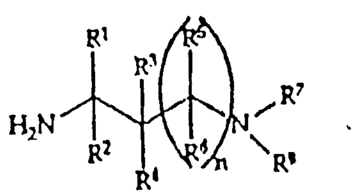

III

dans laquelle les substituants $R^1$ à $R^8$ ont les significations données dans la revendication 1, ou
b) on fait réagir des dérivés d'alkylaminoquinoléines de formule générale

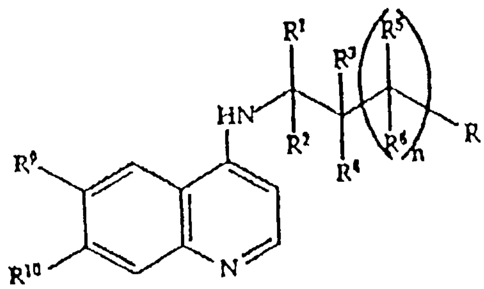

IV

dans laquelle $R^1$ à $R^6$ et $R^9$ et $R^{10}$ ont les significations données dans la revendication 1, et R représente un groupe partant, avec des amines de formule

$$HNR^7R^8 \qquad V,$$

dans laquelle $R^7$ et $R^8$ ont les significations données dans la revendication 1, et

c) si on le désire, on convertit un composé basique de formule I, à l'aide d'un acide, en un sel pharmaceutiquement acceptable.

**12.** Composés selon la revendication 10, pour utilisation en tant que substances actives thérapeutiques.

**13.** Composés selon la revendication 10, pour utilisation en tant que substances actives contre des agents pathogènes paludéens aussi bien résistants à la chloroquine que sensibles à la chloroquine.

**14.** Utilisation des composés selon la revendication 10, dans la lutte contre des maladies ou dans l'amélioration de la santé.

**15.** Médicament contenant un ou plusieurs composés selon la revendication 10 et un excipient thérapeutiquement inerte.

**16.** Médicament selon la revendication 15, pour le traitement ou la prévention du paludisme.